(19) Europäisches Patentamt
European Patent Office
Office européen des brevets

(11) EP 1 670 779 B1

(12) **EUROPÄISCHE PATENTSCHRIFT**

(45) Veröffentlichungstag und Bekanntmachung des Hinweises auf die Patenterteilung:
**24.11.2010 Patentblatt 2010/47**

(21) Anmeldenummer: **04765718.4**

(22) Anmeldetag: **30.09.2004**

(51) Int Cl.:
***C07D 333/16*** *(2006.01)*

(86) Internationale Anmeldenummer:
**PCT/EP2004/010939**

(87) Internationale Veröffentlichungsnummer:
**WO 2005/033094 (14.04.2005 Gazette 2005/15)**

(54) **VERFAHREN ZUR HERSTELLUNG VON 3-METHYLAMINO-1-(THIEN-2-YL)-PROPAN-1-OL**

METHODS FOR THE PRODUCTION OF 3-METHYLAMINO-1-(THIENE-2-YL)-PROPANE-1-OL

PROCEDES POUR PRODUIRE DU 3-METHYLAMINO-1-(THIEN-2-YL)-PROPAN-1-OL

(84) Benannte Vertragsstaaten:
**AT BE BG CH CY CZ DE DK EE ES FI FR GB GR HU IE IT LI LU MC NL PL PT RO SE SI SK TR**

(30) Priorität: **01.10.2003 DE 10345772**

(43) Veröffentlichungstag der Anmeldung:
**21.06.2006 Patentblatt 2006/25**

(73) Patentinhaber: **BASF SE**
**67056 Ludwigshafen (DE)**

(72) Erfinder:
- **STÜRMER, Rainer**
  **67127 Rödersheim-Gronau (DE)**
- **KESSELER, Maria**
  **68167 Mannheim (DE)**
- **HAUER, Bernhard**
  **67136 Fussgönheim (DE)**
- **FRIEDRICH, Thomas**
  **64283 Darmstadt (DE)**
- **BREUER, Michael**
  **67117 Limburgerhof (DE)**

(56) Entgegenhaltungen:
**DE-A- 10 248 479 DE-A- 10 248 480**

- **PATENT ABSTRACTS OF JAPAN Bd. 2003, Nr. 11, 5. November 2003 (2003-11-05) & JP 2003 192681 A (MITSUBISHI RAYON CO LTD), 9. Juli 2003 (2003-07-09)**
- **WHEELER W J ET AL: "AN ASYMMETRIC SYNTHESIS OF DULOXETINE HYDROCHLORIDE, A MIXED UPTAKE INHIBITOR FOR SEROTONIN AND NOREPHINEPHRINE, AND ITS C-14 LABELED ISOTOPOMERS" JOURNAL OF LABELLED COMPOUNDS AND RADIOPHARMACEUTICALS, SUSSEX, GB, Bd. 36, Nr. 3, 1995, Seiten 213-224, XP009019756 ISSN: 0362-4803 in der Anmeldung erwähnt**
- **KAMAL A ET AL: "Chemoenzymatic synthesis of duloxetine and its enantiomer: lipase-catalyzed resolution of 3-hydroxy-3-(2-thienyl) propanenitrile" TETRAHEDRON LETTERS, ELSEVIER SCIENCE PUBLISHERS, AMSTERDAM, NL, Bd. 44, Nr. 25, 16. Juni 2003 (2003-06-16), Seiten 4783-4787, XP004426893 ISSN: 0040-4039 in der Anmeldung erwähnt**
- **LIU H ET AL: "CHEMO-ENZYMATIC SYNTHESIS OF THE ANTIDEPRESSANT DULOXETINE AND ITS ENANTIOMER" CHIRALITY, WILEY-LISS, NEW YORK, US, Bd. 12, Nr. 1, 2000, Seiten 26-29, XP009000316 ISSN: 0899-0042 in der Anmeldung erwähnt**
- **HUMMEL W: "NEW ALCOHOL DEHYDROGENASES FOR THE SYNTHESIS OF CHIRAL COMPOUNDS" 1997, ADVANCES IN BIOCHEMICAL ENGINEERING, BIOTECHNOLOGY, SPRINGER, BERLIN, DE, PAGE(S) 145-184 , XP000677754 ISSN: 0724-6145 das ganze Dokument**
- **DATABASE EMBL [Online] 14. Februar 2003 (2003-02-14), "Lactobacillus brevis radh gene for R-specific alcohol dehydrogenase" XP002339858 Database accession no. AJ544275 & DATABASE UniProt 1. Juni 2003 (2003-06-01), "R-specific alcohol dehydrogenase" XP002339859 Database accession no. Q84EX5**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 670 779 B1

- DATABASE EMBL [Online] 9. August 2001 (2001-08-09), "Sequence 7 from patent US 6225099" XP002339860 Database accession no. AR148418 & DATABASE USPTO PROTEINS [Online] 8. August 2001 (2001-08-08), "Sequence 8 from patent US 6225099" XP002339861 Database accession no. AAE68828
- DATABASE GENESEQ [Online] 31. August 2001 (2001-08-31), "DNA encoding Candida magnoliae carbonyl reductase" XP002339862 Database accession no. AAH27641 & DATABASE GENESEQ [Online] 31. August 2001 (2001-08-31), "Candida magnoliae carbonyl reductase" XP002339863 Database accession no. AAB97187
- ANGEWANDTE CHEMIE (INTERNATIONAL ED. IN ENGLISH), Bd. 43, Nr. 7, 6. Februar 2004 (2004-02-06), Seiten 788-824, XP002339848

**Beschreibung**

Die vorliegende Erfindung beschreibt ein Verfahren zur Herstellung von 3-Methylamino-1-(thien-2-yl)-propan-1-ol der Formel I

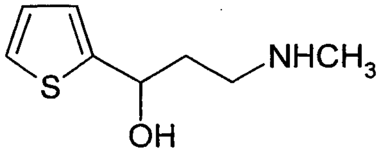

(I)

und insbesondere ein Verfahren zur Herstellung des S-Enantiomers I-S.

Das S-Enantiomer des Aminopropanols I der Formel I-S

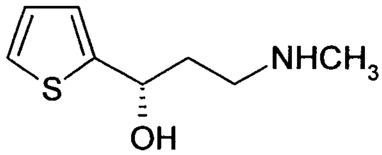

(I-S)

ist eine wichtige Vorstufe für die Synthese des Antidepressivums Duloxetine der Formel II

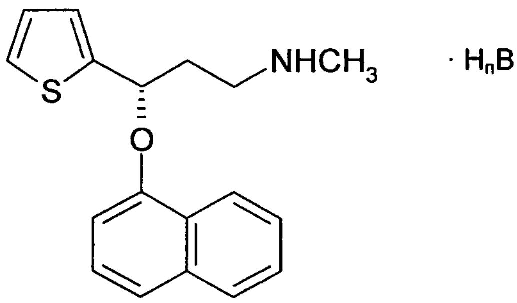

(II)

worin B für einen n-fach negativ geladenen anorganischen oder organischen Säurerest steht und $H_nB$ für eine pharmazeutisch verträgliche Säure.

Verfahren des Standes der Technik zur Herstellung von Duloxetine bzw. ihrer korrespondierenden Base sind aufwendig und erfordern den Einsatz chiraler Reagenzien oder chiraler Edukte.

So beschreibt die EP-B-0273658 ein Verfahren zur Herstellung der korrespondierenden Base von Duloxetine durch Umsetzung von 2-Acetylthiophen in einer Mannich-Reaktion mit Formaldehyd und Dimethylamin, Reduktion der Ketogruppe der dabei erhaltenen Mannich-Base zum racemischen (S)-3-N,N-Dimethylamino-1-(thien-2-yl)propan-1-ol, Veretherung der Alkoholfunktion mit Naphthylfluorid und schließlich Umwandlung der Dimethylamino-Gruppe in eine Methylamino-Funktion. Das gewünschte Enantiomer des Naphtylethers erhält man durch Einsatz chiraler Ausgangsmaterialien oder durch Racemattrennung auf der Stufe des Endprodukts, beispielsweise über die Salze mit optisch aktiven Säuren oder Chromatographie an einer chiralen stationären Phase.

Die US-5,362,886 beschreibt ein analoges Verfahren, bei dem das nach Reduktion der Ketogruppe erhaltene racemische Propanol mit S-Mandelsäure versetzt wird. Das hierbei erhaltene S-Enantiomer des Alkohols wird in die nachfolgenden Reaktionsstufen eingesetzt.

Die EP-A-0457559 beschreibt ebenfalls ein der EP-B-0273658 analoges Verfahren. Hierbei wird die Ketogruppe der Mannich-Base mit dem asymmetrischen Reduktionssystem LAH-Icb (Lithiumaluminiumhydrid-[(2R,2S)-(-)-4-Dimethylamino-1,2-diphenyl-3-methyl-2-butanol]) zum Alkohol in Form des S-Enantiomers reduziert. Nachteilig hierbei ist neben den Kosten die Empfindlichkeit des Reduktionssystems LAH-Icb, das nur wenige Minuten stabil ist.

Auch W. J. Wheeler und F. Kuo beschreiben in Journal of Labelled Compounds and Radiopharmaceuticals, Band XXXVI, Nr. 3, Seite 213 bis 223 ein Verfahren zur Herstellung von Duloxetine. Hierzu wird Thiophen-2-carbonsäurechlorid in einer Stille-Kopplung mit Vinyl-tri-n-butylstannan in Gegenwart katalytischer Mengen Benzylchlor-bis(triphenylphosphin)palladium(II) in DMPU (Dimethylpropylenharnstoff) zu 1-(Thien-2-yl)-propenon der Formel II

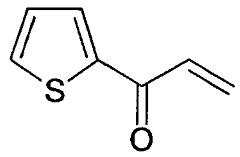

(II)

umgesetzt, welches anschließend durch Behandlung mit Chlorwasserstoff in 3-Chlor-1-(thien-2-yl)-propan-1-on der Formel III.1

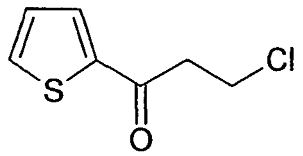

(III.1)

überführt wird. Das so erhaltene Chlorpropanon III.1 wird anschließend unter Verwendung eines chiralen Oxazaborolidins und $BH_3$ zu (S)-3-Chlor-1-(thien-2-yl)- propan-1-ol der Formel IV.1-S

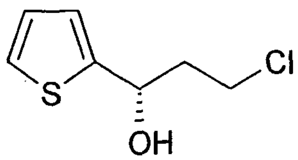

(IV.1-S)

reduziert. Der so erhaltene Alkohol IV.1-S wird durch sukzessive Umsetzung mit Natriumiodid und anschließend mit Methylamin in (S)-3-Methylamino-1-(thien-2-yl)-propan-1-ol I-S überführt. Durch nachfolgende sukzessive Umsetzung mit Natriumhydrid, 1-Fluornaphtalin und Chlorwasserstoff erhält man Duloxetine in Form des Hydrochlorids. Von Nachteil ist hierbei zum einen, dass zur Herstellung des Chlorpropanon-Zwischenprodukts III.1 zahlreiche Schritte und teure Reagenzien erforderlich sind. Zum anderen wird bei der Überführung des Chlorpropanons III.1 in den Aminoalkohol das Chlorpropanol IV.1-S isoliert. Untersuchungen der Anmelderin haben jedoch gezeigt, dass dieses Chlorpropanol empfindlich ist und sich in einer stark exothermen Reaktion leicht zersetzt, was nicht nur zu Ausbeuteverlusten bezüglich des Aminoalkohols führt, sondern auch die Handhabung der Reaktion in technischem Maßstab erschwert.

Verfahren zur Herstellung des von W. J. Wheeler et al. beschriebenen, bei der Synthese von Duloxetine als Zwischenprodukt auftretenden 3-Chlor-1-(thien-2-yl)-propan-1-ons sind literaturbekannt. Nachteilig bei den bekannten Verfahren des Standes der Technik ist jedoch, dass entweder das Chlorpropanon III.1 in schlechter Ausbeute anfällt oder mit schwierig handzuhabenden Reagenzien gearbeitet werden muss. So beschreiben A. Etienne et al. in CR Acad. Sci., Ser. C, 1979, 288 (1), 49-52, die Herstellung des Chlorpropanons III.1 durch Friedel-Crafts-Reaktion von Thiophen mit 3-Chlorpropionylchlorid in Gegenwart von Aluminiumtrichlorid als Lewissäure-Katalysator und in Nitromethan als Lösungsmittel. Das Chlorpropanon III.1 wird in einer Ausbeute von nur 7% erhalten. Auch die entsprechende, von Liu et al. in Chirality, 12, 26-29 (2000), beschriebene Umsetzung in Gegenwart von Zinntetrachlorid als Lewissäure-Katalysator und Benzol als Lösungsmittel führt zu unbefriedigenden Ausbeute. Meth-Cohn et al. beschreiben in Acta Chem. Scand. B 20 (6), 1577-1587 (1966) die entsprechende Friedel-Crafts-Acylierung an Thiophen in Gegenwart von Eisentrichlorid bzw. Aluminiumtrichlorid, wobei das Chlorpropanon III.1 in mäßigen bis guten Ausbeuten entsteht. Nachteilig ist hierbei, dass als Lösungsmittel Schwefelkohlenstoff verwendet werden muss.

Kamal, G. B. R. Khanna, R. Ramu und T. Krishnaji beschreiben in Tetrahedron Lett. 44, 2003, 4783-4787 die Herstellung der Duloxetine-Vorstufe (S)-3-Hydroxy-3-(thien-2-yl)-propannitril durch Acetylierung von Thiophen mit Chloracetylchlorid, Reduktion des Ketons mit Natriumborhydrid zum racemischen Alkohol, Substitution des Chlorrestes durch Cyanid und Umsetzung des racemischen Nitrilalkohols mit Vinylacetat in Gegenwart einer Lipase aus Pseudomonas cepacia (immobilisiert auf Diatomit), wobei die Lipase selektiv die Veresterung des R-Enantiomers katalysiert, so dass das gewünschte S-Enantiomer, welches unverestert bleibt, in reiner Form isoliert werden kann. Von Nachteil ist hierbei zum einen die große Anzahl an erforderlichen Reaktionsschritten und zum anderen der Verlust der Hälfte des Nitrilalkohols, da der veresterte R-Anteil nicht weiter zu Duloxetine umgesetzt wird.

Aufgabe der vorliegenden Erfindung war es daher, ein Verfahren zur Herstellung von 3-Methylamino-1-(thien-2-yl)-propan-1-ol I bereitzustellen, welches die hier geschilderten Nachteile des Standes der Technik überwindet. Zudem sollte es die Verbindung I in guter Gesamtausbeute liefern und insbesondere auch die enantioselektive Herstellung des S-Enantiomer I-S erlauben.

Die Aufgabe wurde dadurch gelöst, dass man zunächst Thiophen in einer Friedel-Crafts-Reaktion mit einem β-Halogenpropionsäurehalogenid oder einem Acrylsäurehalogenid in Gegenwart einer Lewissäure umsetzt, wobei man vor der Isolierung des Reaktionsprodukts einen Halogenwasserstoff einleitet. Anschließend reduziert man die Ketogruppe des dabei erhaltenen 3-Halogen-1-(thien-2-yl)-propan-1-ons der Formel III

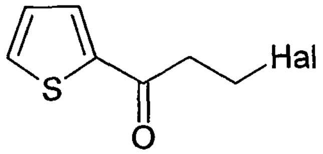

(III)

Hal = Halogen

und setzt das reduzierte Produkt der Formel IV

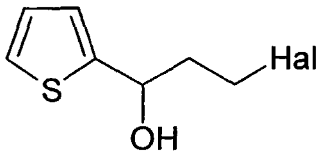

(IV)

mit Methylamin um.

Beschrieben wird daher ein Verfahren zur Herstellung von 3-Methylamino-1-(thien-2-yl)-propan-l-oi der Formel I

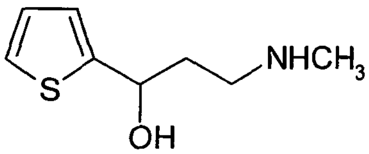

(I)

bei dem man

a) Thiophen mit einem β-Halogenpropionsäurehalogenid oder einem Acrylsäurehalogenid in Gegenwart einer Lewis-Säure zu einem 3-Halogen-1-(thien-2-yl)-propan-1-on umsetzt, wobei man gleichzeitig oder nach erfolgter Umsetzung, jedoch vor der Isolierung des Reaktionsprodukts, ei nen Halogenwasserstoff einleitet und

b) das in Schritt a) erhaltene Propanon reduziert und anschließend, vorzugsweise ohne Isolierung des Reaktionsprodukts, mit Methylamin umsetzt.

Das erfindungsgemäße Verfahren liefert die Zielverbindung I in guten Gesamtausbeuten. Die Herstellung der Halogenpropanon-Zwischenstufe III ist zudem nicht an den Einsatz teurer zinnorganischer Reagenzien gekoppelt. Das schwierig handzuhabende Halogenpropanol IV braucht nicht isoliert zu werden. Zudem erlaubt das Verfahren in einfacher Weise eine enantioselektive Herstellung des S-Enantiomers I-S, indem man die Reduktion der Halogenpropenon-Zwischenstufe III in Gegenwart eines chiralen Katalysators oder eines chiralen Reduktionsmittels durchführt, welche eine Selektivität bezüglich der Bildung von (S)-3-Halogen-1-(thien-2-yl)-propan-l-ol I-S aufweisen.

"Enantioselektivität" im Rahmen der vorliegenden Erfindung bedeutet, dass der Enantiomerenüberschuss ee (in %) des S-Enantiomers, der sich in bekannter Weise berechnet nach:

$$ee\ (\%) = [\text{S-Enantiomer} - \text{R-Enantiomer} / (\text{S-Enantiomer} - \text{R-Enantiomer})] \times 100$$

wenigstens 50 %, vorzugsweise wenigstens 80 %, insbesondere wenigstens 90 % und speziell wenigstens 95 % beträgt.

Durch das Einleiten eines Halogenwasserstoffs während oder vorzugsweise nach erfolgter Acylierung, jedoch vor der Isolierung des Reaktionsprodukts, erhält man als Endprodukt des Schrittes a) im Wesentlichen kein 1 -Thien-2-yl-propenon II, welches bei den Verfahren des Standes der Technik stets als Nebenprodukt auftritt und die Ausbeute an gewünschtem Acylierungsprodukt III schmälert.

Als Lewis-Säure kommen kovalente Metallhalogenide und Halbmetallhalogenide, die eine Elektronenpaarlücke aufweisen, in Betracht. In der Regel sind sie ausgewählt unter Halogenverbindungen des Titans, Zinns, Aluminiums, Vanadiums, Eisens oder Bors. Bevorzugt sind die Chloride; im Falle des Aluminiums sind jedoch auch die Monoalkylaluminiumdichloride und die Dialkylaluminiumchloride geeignet. Im Falle des Bors ist auch das Fluorid geeignet. Beispiele für geeignete Lewis-Säuren sind Titantetrachlorid, Bortrichlorid, Bortrifluorid, Zinntetrachlorid, Vanadiumpentachlorid, Eisentrichlorid, Aluminiumtrichlorid, Alkylaluminiumdichloride und Dialkylaluminiumchloride. Besonders bevorzugt verwendet man Aluminiumtrichlorid.

Geeignete β-Halogenpropionsäurehalogenide sind 3-Chlorpropionsäurechlorid und 3-Brompropionsäurebromid oder -chlorid. Vorzugsweise verwendet man 3-Chlorpropionsäurechlorid.

Als Acrylsäurehalogenid wird vorzugsweise Acrylsäurechlorid verwendet.

Vorzugsweise setzt man in Schritt a) als Acylierungsmittel ein β-Halogenpropionsäurehalogenid ein.

Geeignete Halogenwasserstoffe sind Chlorwasserstoff und Bromwasserstoff. Vorzugsweise setzt man einen Halogenwasserstoff ein, in welchem das Halogenatom dem β-Halogenrest im eingesetzten β-Halogenpropionsäurehalogenid entspricht. Dementsprechend verwendet man bei Einsatz von 3-Chlorpropionsäurechlorid vorzugsweise Chlorwasserstoff.

Als Lösungsmittel für die Umsetzung in Schritt a) sind sämtliche Lösungsmittel geeignet, die üblicherweise in Friedel-Crafts-Acylierungsreaktionen eingesetzt werden. Geeignet sind grundsätzlich aprotische Lösungsmittel, welche die Reaktivität der eingesetzten Reagenzien, insbesondere der Lewis-Säure, nicht herabsetzen bzw. mit der Lewis-Säure unter den gegebenen Reaktionsbedingungen keine Konkurrenzreaktionen eingehen. Beispiele hierfür sind der zu acylierende Aromat selbst (d.h. Thiophen), aromatische Kohlenwasserstoffe, die im Vergleich zu Thiophen deutlich schwerer acyliert werden, wie Benzol, Nitrobenzol und halogenierte aromatische Kohlenwasserstoffe, z.B. Chlorbenzol und Dichlorbenzol, des weiteren halogenierte aliphatische Kohlenwasserstoffe, insbesondere Halogenalkane, wie Chlormethan, Dichlormethan, Chloroform, Tetrachlorkohlenstoff, Chlorethan, Di- und Trichlorethan. Geeignet sind auch Gemische der vorstehend genannten Lösungsmittel. Vorzugsweise verwendet man Lösungsmittel, in denen die Friedel-Crafts-Acylierung im Wesentlichen homogen ablaufen kann, wie Nitrobenzol oder halogenierte Kohlenwasserstoffe. Bevorzugt sind insbesondere halogenierte aromatische oder aliphatische Kohlenwasserstoffe, wobei Halogenalkane besonders bevorzugt sind. Speziell verwendet man Dichlorethan oder Chlorbenzol.

Die Lewis-Säure muss in wenigstens äquimolaren Mengen, bezogen auf die theoretisch zu erreichende Menge an acyliertem Thiophen, eingesetzt werden, da sie mit dem bei der Acylierung gebildeten Keton einen Komplex bildet, der unter den üblichen Reaktionsbedingungen der Friedel-Crafts-Reaktion stabil ist. Vorzugsweise wird sie in einem Molverhältnis von 1:1 bis 1:2, besonders bevorzugt von 1:1 bis 1:1,5 und insbesondere von 1:1,1 bis 1:1,5, bezogen auf 1 Mol der in geringerem Anteil eingesetzten Acylierungskomponente (Thiophen oder β-Halogenpropionsäurehalogenid) eingesetzt.

Thiophen und das β-Halogenpropionsäurehalogenid werden in einem Molverhältnis von vorzugsweise 1:0,5 bis 1:2, besonders bevorzugt von 1:0,7 bis 1:1,5 und insbesondere von 1:0,8 bis 1:1,2 eingesetzt.

Die Reihenfolge, in welcher bei der Friedel-Crafts-Acylierung die Reagenzien zusammengegeben werden, ist von untergeordneter Bedeutung. So kann man beispielsweise die Lewis-Säure im Lösungsmittel vorlegen und zuerst das β-Halogenpropionsäurehalogenid und anschießend das Thiophen zugeben. Alternativ kann man Thiophen und Lewis-Säure vorlegen und mit dem β-Halogenpropionsäurehalogenid versetzen.

In der Regel ist es günstig, bei der Zugabe des β-Halogenpropionsäurehalogenids zu kühlen, da die Reaktion der Lewis-Säure mit dem Säurehalogenid in der Regel stark exotherm verläuft. Die Reaktionstemperatur wird u.a. in Abhängigkeit vom Lösungsmittel gewählt. Üblicherweise liegt sie im Bereich von 10 °C bis 40 °C. Bei Verwendung von Halogenkohlenwasserstoffen, insbesondere von Halogenalkanen beträgt die Reaktionstemperatur geeigneterweise höchstens 50 °C, da ansonsten das Lösungsmittel selbst in Reaktion tritt. Vorzugsweise beträgt die Reaktionstemperatur -20 °C bis 40 °C, besonders bevorzugt 0 bis 30 °C.

Der Reaktionsdruck ist prinzipiell nicht kritisch. Im Allgemeinen wird die Reaktion bei Normaldruck durchgeführt; sie kann jedoch auch bei Über- oder Unterdruck erfolgen. Überdruck wird beispielsweise angewendet, wenn das Lösungsmittel leichtflüchtig ist oder unter Normalbedingungen nicht flüssig ist, wie dies beispielsweise bei Chlormethan der Fall ist.

Die Einleitung des Halogenwasserstoffs erfolgt vorzugsweise nach erfolgter Acylierung. Die Beendigung der Acylierungsreaktion kann nach üblichen Verfahren des Standes der Technik festgestellt werden, z.B. durch Gaschromatographie, Dünnschichtchromatographie oder NMR-Spektroskopie. Die Dauer der Einleitung hängt unter anderem von der Ansatzgröße ab und kann vom Fachmann im Einzelfall bestimmt werden. In der Regel erfolgt sie mindestens

so lange, bis kein 1-Thien-2-yl-propenon mehr nachgewiesen werden kann.

Zur Aufarbeitung des Acylierungsprodukts wird das Reaktionsgemisch in der Regel zunächst hydrolytisch aufgearbeitet, um den gebildeten Keton-Lewis-Säure-Komplex zu spalten. Zur Hydrolyse verwendet man üblicherweise Wasser oder auch verdünnte wässrige Mineralsäuren, z.B. verdünnte Salzsäure. Die weitere Reinigung und Isolierung erfolgt nach bekannten Verfahren, wie sie beispielsweise in Organikum, VEB Deutscher Verlag der Wissenschaften, 17. Auflage, 1988, S. 323 ff. beschrieben sind.

Das erfindungsgemäße Verfahren führt in Schritt a) zu einem 3-Halogen-(thien-2-yl)-1-propanon III in hohen Ausbeuten. Im Verlauf der Acylierungsreaktion gegebenenfalls gebildetes 1-Thien-2-yl-propenon II wird im wesentlichen vollständig in das 3-Halogen-(thien-2-yl)-1-propanon III überführt, so dass das Reaktionsgemisch höchstens 1 Mol-%, besonders bevorzugt höchstens 0,5 Mol-% Propenon II, bezogen auf die Ausbeute an 3-Halogen-(thien-2-yl)-1-propanon III, enthält.

Die Reduktion des Propanons III in Schritt b) gelingt beispielsweise mit einem Metall- oder Halbmetallhydrid oder mit Wasserstoff in Gegenwart eines geeigneten Übergangsmetallkatalysators.

Geeignete Metall- oder Halbmetallhydride sind sowohl die neutralen als auch die komplexen Hydride von Metallen bzw. Halbmetallen. Günstigerweise verwendet man Metall- oder Halbmetallhydride, die sich bei der Reduktion von Ketonen zu Alkoholen bewährt haben. Vorzugsweise handelt es sich um die Hydride des Bors oder des Aluminiums.

Geeignete neutrale Hydride sind beispielsweise Boran ($BH_3$; $B_2H_6$), Alan ($AlH_3$), Silan ($SiH_4$), Mono- und Dialkylborane, wie Bis(3-methylbut-2-yl)boran (Disiamylboran) oder 9-Borabicyclo[3.3.1]nonan (9-BBN), Mono- und Dialkylaluminiumverbindungen, wie Diisobutylaluminiumhydrid (DIBAL-H), und Trialkylsilane, wie Trimethylsilan oder Triethylsilan.

Geeignete komplexe Hydride sind beispielsweise komplexe Borhydride, wie Natriumborhydrid ($NaBH_4$), Lithiumborhydrid ($LiBH_4$) oder Calciu mborhydrid $Ca[BH_4]_2$, komplexe Aluminiumhydride, wie Lithiumaluminiumhydrid ($LiAlH_4$), komplexe Alkyl- oder Alkoxyborhydride, wie Lithiumtriethylborhydrid oder Kaliumtriisopropoxyborhydrid ($KB[OCH(CH_3)_2]_3H$), oder komplexe Alkyl- oder Alkoxyaluminiumhydride, wie Natriumdiethylaluminiumhydrid, Lithiumbis (2-methoxyethoxy)aluminiumhydrid ($Li\text{-}Al[OC_2H_4OCH_3]_2H_2$), Natriumbis(2-methoxyethoxy)aluminiumhydrid ($NaAl[OC_2H_4OCH_3]_2H_2$; "RedAl") oder Lithiumaluminiumtris(tert-butoxy)hydrid ($Li\text{-}Al[OC(CH_3)_3]_3H$) und dergleichen.

Für die Reduktion mit Metall- oder Halbmetallhydriden geeignete Lösungsmittel hängen insbesondere vom verwendeten Reduktionsmittel ab und sollten selbstverständlich keine Gruppen enthalten, die unter den Reaktionsbedingungen ebenfalls reduziert werden. So führt man die Reduktion mit den vorstehend genannten Hydriden vorzugsweise in aprotischen Lösungsmitteln ohne funktionelle Gruppen, die unter den gegebenen Reaktionsbedingungen reduzierbar sind, durch. Beispiele hierfür sind aromatische und aliphatische Kohlenwasserstoffe, beispielsweise $C_5$-$C_8$-Alkane und $C_5$-$C_8$-Cycloalkane, wie Pentan, Hexan, Heptan, Cyclopentan, Cyclohexan und Cyclooctan, Aromaten, wie Benzol, Toluol, Nitrobenzol, Chlor- und Dichlorbenzol, weiterhin offenkettige und cyclische Ether mit 4 bis 8 Kohlenstoffatomen, wie Diethylether, Methyltert-butylether, Tetrahydrofuran oder Dioxan, ferner chlorierte Kohlenwasserstoffe, insbesondere Halogenalkane, wie Chlormethan, Dichlormethan, Chloroform, Tetrachlorkohlenstoff, Di- oder Trichlorethan. Geeignet sind auch Gemische der vorstehend genannten Lösungsmittel. Vorzugsweise verwendet man die vorstehend genannten aromatischen Kohlenwasserstoffe, Ether oder Halogenkohlenwasserstoffe.

Die Reduktion mit einigen der oben genannten komplexen Hydride, insbesondere mit weniger reaktiven Hydriden, wie Natriumborhydrid, gelingt auch in Gegenwart protischer Lösungsmittel, z.B. von $C_1$-$C_3$-Alkoholen, wie Methanol, Ethanol, Propanol oder Isopropanol, oder sogar in wässriger Lösung. In diesem Fall verwendet man vorzugsweise ein Gemisch aus wenigstens einem der zuvor genannten aprotischen Lösungsmittel und wenigstens einem Alkohol. Da die Hydride in basischen protischen Lösungen stabiler sind, erfolgt die Umsetzung bei Verwendung protischer Lösungsmittel vorzugsweise in Gegenwart einer geeigneten Base. Geeignete Basen sind beispielsweise Alkalimetallhydroxide, wie Natrium- oder Kaliumhydroxid, oder Alkylimetallcarbonate, wie Natrium- oder Kaliumcarbonat. Vorzugsweise verwendet man Natriumhydroxid.

Die Reduktion mit vielen der zuvor genannten Metall- oder Halbmetallhydride verläuft häufig exotherm, so dass die Umsetzung geeigneterweise unter Abführung der Reaktionswärme, d.h. unter Kühlung durchgeführt wird. Die Reaktionstemperatur beträgt vorzugsweise -50 bis 40 °C, besonders bevorzugt -30 bis 30 °C und insbesondere - 20 bis 20 °C. Die Reduktion kann nach üblichen Verfahren des Standes der Technik durchgeführt werden, wie sie beispielsweise in Organikum, VEB Deutscher Verlag der Wissenschaften, 17. Auflage, 1988, S. 492 ff. beschrieben sind. Dabei legt man in der Regel das Propanon III vor und gibt das Reduktionsmittel portionsweise zu. Es ist jedoch auch möglich, Propanon III und Reduktionsmittel gleichzeitig portionsweise zuzugeben.

Die Reduktion des Propanons III gelingt beispielsweise auch mit einem Aluminiumalkoholat. Die Reduktion von Ketonen mit einem Aluminiumalkoholat bezeichnet man üblicherweise auch als Meerwein-Ponndorf-Verley-Reduktion. Dabei wird ein Keton in den entsprechenden Alkohol überführt und das Alkoholat wird gleichzeitig zum entsprechenden Aldehyd (bei Alkoholaten primärer Alkohole) bzw. Keton (bei Alkoholaten sekundärer Alkohole) oxidiert. Die Durchführung der Reaktion kann nach bekannten Verfahren erfolgen, wie sie beispielsweise in Organikum, VEB Deutscher Verlag der Wissenschaften, 17. Auflage, 1988, S. 486 ff. beschrieben sind.

Die Reduktion des Propanons III gelingt außerdem auch durch katalytische Hydrierung, d.h. durch Umsetzung des Propanons III mit Wasserstoff in Gegenwart geeigneter Übergangsmetallkatalysatoren. Zu den geeigneten Übergangsmetallen zählen die Metalle der Nebengruppen VIII, VI und I, insbesondere Platin, Ruthenium, Kupfer, Chrom und Nickel. Die Katalysatoren müssen selbstverständlich so gewählt werden, dass sie die Hydrierung der Thiophengruppe nicht katalysieren. Die Katalysatoren können sowohl als Heterogen- als auch als Homogenkatalysatoren eingesetzt werden. Geeignete Verfahren sind prinzipiell bekannt und beispielsweise in Transition Metals in Organic Synthesis. M. Beller, C. Bolm, Wiley-VCH, Weinheim, 1998, Band 2, Seite 1 ff. (Homogenkatalysatoren) bzw. Seite 81 ff. (Heterogenkatalysatoren) beschrieben.

Die Reduktion in Schritt b) erfolgt vorzugsweise mit einem Metall- oder Halbmetallhydrid und besonders bevorzugt mit einem der vorstehen genannten komplexen Metall- oder Halbmetallhydride. Speziell setzt man Natriumborhydrid ein.

Mit den vorstehend beschriebenen nicht asymmetrischen Reduktionsmitteln wird das prochirale 3-Halogen-1-(thien-2-yl)-propan-1-on III vornehmlich zu dem racemischen Alkohol IV reduziert. Die nachfolgende Umsetzung mit Methylamin führt entsprechend zum racemischen 3-Methylamino-1-(thien-2-yl)-propan-1-ol I.

In einer bevorzugten Ausführungsform dient das erfindungsgemäße Verfahren zur Herstellung von (S)-3-Methylamino-1-(thien-2-yl)-propan-1-ol der Formel I-S

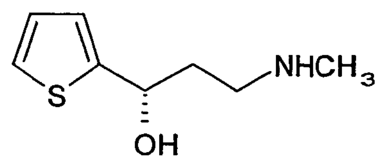

(I-S)

bzw. des Gemischs mit seinem R-Enantiomer I-R, wobei das Enantiomer I-S im Gemisch überwiegt, wobei man die Reduktion in Schritt b) in Gegenwart eines chiralen Reduktionsmittels oder eines chiralen Katalysators durchführt, welche eine Selektivität bezüglich der Bildung von (S)-3-Methylamino-1-(thien-2-yl)propan-1-ol I-S aufweisen.

Hierbei wird das prochirale Halogenpropanon III in Schritt b) enantioselektiv zum S-Halogenpropanol der Formel IV-S

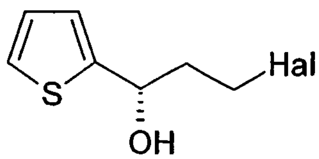

(IV-S)

bzw. zu einem Gemisch aus S- und R-Enantiomer, in welchem das S-Enantiomer überwiegt, reduziert. Zu diesem Zweck setzt man in Schritt b) als Reduktionsmittel beispielsweise ein asymmetrisches Metall- oder Halbmetallhydrid oder führt die Reduktion in Gegenwart einer asymmetrische Induktion vermittelnden Verbindung durch.

Bei den asymmetrischen Metall- oder Halbmetallhydriden handelt es sich vorzugsweise um asymmetrische Aluminium-, Bor- oder Siliciumhydride. Geeignete asymmetrische Borhydride sind beispielsweise in E. J. Corey, C. J. Helal, Angew. Chem. 1998, 110, 2092-2118 oder in M. M. Midland, L. A. Morell, Methoden Org. Chem., Houben-Weyl, 4. Aufl., Band E 21 d, S. 4082-4098, 1995 beschrieben, worauf hiermit in vollem Umfang Bezug genommen wird. Geeignete asymmetrische Siliciumhydride sind unter anderem in H. Brunner, Methoden Org. Chem., Houben-Weyl, 4. Aufl., Band E21d, S. 4074-4081, 1995 beschrieben, worauf ebenfalls in vollem Umfang Bezug genommen wird.

Unter asymmetrische Induktion vermittelnde Verbindungen versteht man im Rahmen der vorliegenden Erfindung solche, die durch Beeinflussung des eigentlichen Reduktionsmittels, z.B. durch Bindungsbildung oder Komplexbildung mit dem Reduktionsmittel oder durch Aufnahme eines Wasserstoffatoms oder eines anderen reduktiven Bestandteils aus dem Reduktionsmittel, die Reduktion des Propanons III enantioselektiv gestalten. Die asymmetrische Induktion vermittelnden Verbindungen selbst sind in der Regel nicht reduzierend.

Hierzu gehören zum einen asymmetrische Hydrierkatalysatoren. Bei der asymmetrischen Hydrierung dient Wasserstoff als eigentliches Reduktionsmittel, während der asymmetrische Katalysator die enantioselektive Bildung eines der möglichen Enantiomere begünstigt. Die Hydrierung kann sowohl in heterogener als auch in homogener Phase erfolgen. Geeignete Katalysatoren für die asymmetrische Hydrierung in heterogener Phase sind beispielsweise in A. Baiker, H. U. Blaser in Handbook of Heterogeneous Catalysis, Band 5 (Herausgeber G. Ertl, H. Knörzinger, J.Weitkamp),

Wiley-VCH, Weinheim, 1997, 2422-2436, auf die hiermit in vollem Umfang Bezug genommen wird, beschrieben.

Von größerer Bedeutung sind jedoch Katalysatoren für die Hydrierung in homogener Phase. Besonders geeignet sind Katalysatoren, die ein Metall der Nebengruppe VIII, z.B. Platin oder Ruthenium, insbesondere Ruthenium, und wenigstens einen phosphorhaltigen Liganden enthalten. Geeignete Katalysatoren sind beispielsweise in R. Noyori, T. Ohkuma, Angew. Chem. 2001, 113, 40-75 beschrieben, auf die hiermit in vollem Umfang Bezug genommen wird. Insbesondere sind Ruthenium(II)-Diaminkomplexe geeignet, in denen das Ruthenium außerdem an einen zweizähnigen chiralen Diphosphanliganden gebunden ist. Beispiele für Diphosphanliganden, die für die Reduktion des Halogenpropanons III zum S-Halogenpropanol IV-S geeignet sind, sind (R)-BINAP der Formel A, (R,R)-DIOP der Formel B und (R, R)-CHIRAPHOS der Formel C

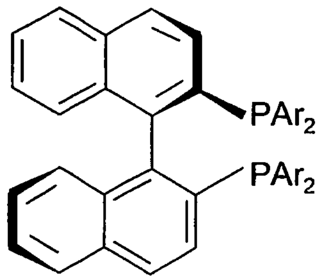

A

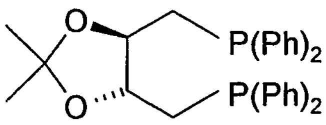

B

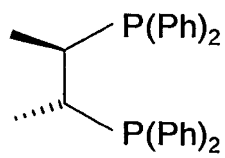

C

Ar = Phenyl (BINAP)
4-Methylphenyl (TolBINAP)
3,5-Dimethylphenyl (XylBINAP)

Ph = Phenyl

Außerdem enthalten besonders bevorzugte Katalysatoren einen Diaminliganden. Beispiele für geeignete Diaminliganden sind 1,2-Ethylendiam in, 1,2-Diphenyl-1,2-Ethylendiamin und 1,2-Cyclohexandiamin in ihren enantiomeren Formen.

Die Hydrierung erfolgt in der Regel unter den zuvor beschriebenen Bedingungen.

Eine besonders bevorzugte asymmetrische Induktion vermittelnde Verbindung ist das Oxazaborylidin der Formel D

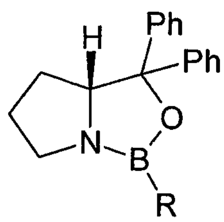

(D)

worin R für $C_1$-$C_4$-Alkyl, wie Methyl, Ethyl, Propyl, Isopropyl, n-Butyl, sec-Butyl, Isobutyl oder tert-Butyl und insbesondere für Methyl steht.

Mit diesem Reagenz sind prochirale Ketone mit hoher Enantioselektivität in sekundäre Alkohole überführbar (E. J. Corey, Pure Appl. Chem. 62, 1209, 1990; E. J. Corey, J. O. Link, J. Am. Chem. Soc. 114, 1906, 1992). Als eigentliches Reduktionsmittel dienen Borane, z.B. $BH_3$, Dialkylborane, Dialkoxyborane oder Diaryloxyborane. Die Umsetzung des Chlorpropanons III.1 mit dem Oxazaborylidin D (R = Methyl) und $BH_3$ zum S-Chlorpropanol IV.1-S wurde bereits von W. J. Wheeler und F. Kuo in Journal of Labelled Compounds and Radiopharmaceuticals, Band XXXVI, Nr. 3, S. 213-223 beschrieben.

Bei der Reduktion wird das Oxazaborylidin in der Regel in katalytischen Mengen eingesetzt. Das Boran wird in wenigstens äquimolaren Mengen, bezogen auf das Halogenpropanon III, vorzugsweise jedoch im Überschuss verwendet. Die Reaktion erfolgt meist in geeigneten Lösungsmitteln. Geeignete Lösungsmittel sind aprotische Lösungsmittel, die keine unter den gegebenen Reaktionsbedingungen reduzierbare Gruppen besitzen. Hierzu zählen insbesondere die zuvor genannten Halogenalkane, Aromaten und cyclischen oder acyclischen Ether. Bevorzugt verwendet man

Ether, wobei Tetrahydrofuran besonders bevorzugt ist. Die Reaktionstemperatur beträgt vorzugsweise -80 bis 20 °C, besonders bevorzugt -30 bis 10 °C. Die Durchführung erfolgt in der Regel so, dass man das Oxazaborylidin im Lösungsmittel vorlegt und bei der gewünschten Reaktionstemperatur entweder zuerst das Boran und anschließend das Halogenpropanon III oder umgekehrt zuerst das Halogenpropanon III und anschließend das Boran zufügt, wobei der erste Zugabemodus bevorzugt ist. Die Reaktionsdauer ist unter anderem von der Ansatzgröße abhängig und kann im Einzelfall vom Fachmann bestimmt werden.

Es hat sich überraschenderweise gezeigt, dass die enantioselektive Reduktion des Propanons III zum S-Halogenpropanol IV-S auf enzymkatalysierem Weg , insbesondere in Gegenwart einer Dehydrogenase, gut gelingt. In einer weiteren bevorzugten Ausführungsform des erfindungsgemäßen Verfahrens wird daher eine Dehydrogenase als Reduktionsmittel eingesetzt.

Nach beendeter Reduktion wird im erfindungsgemäßen Verfahren je nach verwendetem Reduktionsverfahren der eingesetzte Katalysator bzw. überschüssiges Reduktionsmittel gegebenenfalls desaktiviert bzw. entfernt. Bei der Verwendung von Metall- oder Halbmetallhydriden erfolgt dies in der Regel durch Hydrolyse, beispielsweise mit einer wässrigen oder alkoholischen Lösung. Auch bei der Verwendung homogener Hydrierungskatalysatoren oder bei der Reduktion mit Aluminiumalkoholaten wird häufig hydrolytisch aufgearbeitet. Erfolgt die Reduktion in Form einer katalytischen Hydrierung in heterogener Phase oder mit einem Enzym als Reduktionsmittel, so kann der Katalysator bzw. das Enzym durch physikalische Trennung, z.B. durch Abdekantieren oder Filtration entfernt werden. Bei der Verwendung homogener Reduktionssysteme, insbesondere bei der Reduktion mit Metall- oder Halbmetallhydriden, wird vorzugsweise jedoch zunächst nicht desaktiviert.

Es hat sich als vorteilhaft erwiesen, wenn man nach der Reduktion das erhaltene Halogenpropanol IV nicht isoliert, sondern direkt mit Methylamin zum 3-Methylamino-1-(thien-2-yl)-propan-1-ol I ungesetzt. Hierzu wird das gegebenenfalls desaktivierte bzw. von heterogenen Reduktionssystemen abgetrennte Reaktionsgemisch mit Methylamin versetzt und vorzugsweise bei erhöhter Temperatur, beispielsweise bei 30 bis 80 °C, insbesondere bei 50 bis 70 °C, umgesetzt. Das Methylamin kann entweder gasförmig oder als wässrige Lösung eingesetzt werden, wobei die Verwendung als wässrige Lösung bevorzugt ist. Vorzugsweise wird es in einer Menge von 1 bis 100 Moläquivalenten, besonders bevorzugt von 5 bis 10 Moläquivalenten, bezogen auf die Menge des eingesetzten Halogenpropanon III, eingesetzt. Die Umsetzung erfolgt vorzugsweise bei einem Druck von 1 bis 250 bar und insbesondere unter dem Eigendruck, den das System selbst erzeugt.

Nach erfolgter Umsetzung mit Methylamin wird das Reaktionsgemisch in üblicher Weise aufgearbeitet. Hierzu wird, falls das nicht schon vor der Methylamin-Zugabe erfolgte, der Katalysator bzw. das Reduktionsmittel wie bereits beschrieben desaktiviert und abgetrennt, das Lösungsmittel wird entfernt und aus dem Rückstand wird sauberes Methylaminopropanol beispielsweise durch Kristallisation, Digerieren, Extraktion oder Chromatographie isoliert.

Mit dem erfindungsgemäßen Verfahren ist 3-Methylamino-1-(thien-2-yl)-propan-1-ol I in guten Ausbeuten erhältlich. Insbesondere sind in Schritt a) des Verfahrens keine teuren oder aufwändig handzuhabenden Reagenzien oder Lösungsmittel erforderlich und das Halogenpropanon III entsteht in sehr hohen Ausbeuten. In Schritt b) wird die Isolierung des empfindlichen Halogenpropanols IV und ein damit verbundener Ausbeuteverlust bzw. die schwierige Handhabung des Halogenpropanols IV vermieden. Außerdem ist mit dem erfindungsgemäßen Verfahren das S-Enantiomer des Methylaminopropanols I-S, welches für die weitere Umsetzung zu Duloxetine wesentlich ist, selektiv erhältlich, wenn in Schritt b) die Reduktion mit einem chiralen Reduktionsmittel erfolgt.

Ein weitere Gegenstand der vorliegenden Erfindung betrifft außerdem ein Verfahren zur Herstellung von (S)-3-Methylamino-1-(thien-2-yl)-propan-1-ol der Formel I-S, bei dem man ein 3-Halogen-1-(thien-2-yl)-propan-1-on III enantioselektiv reduziert wird, **dadurch gekennzeichnet, dass** die Reduktion in Gegenwart einer Dehydrogenase erfolgt. In einer bevorzugten Ausführungsform des Verfahrens wird das dabei erhaltene (S)-3-Halogen-1-(thien-2-yl)-propan-1-ol IV-S ohne Isolierung mit Methylamin umgesetzt. Bezüglich geeigneter und bevorzugter Dehydrogenasen und der Durchführung des Verfahrens wird auf die folgenden Ausführungen ausdrücklich verwiesen.

<u>Biochemische Ausführungsformen:</u>

Geeignete Dehydrogenasen (EC 1.1.X.X) sind vor allem Dehydrogenasen (E.C. 1.1.1.x) insbesondere Alkohol Dehydrogenasen (E.C.1.1.1.1 oder E.C.1.1.1.2), welche die selektive Reduktion des Halogenpropanons III zum S-Halogenpropanol IV-S bewirken. Die Dehydrogenase wird bevorzugt aus einem Mikroorganismus, besonders bevorzugt aus einem Bakterium, einem Pilz, insbesondere einer Hefe, jeweils hinterlegt in Stammsammlungen oder erhältlich aus Isolaten natürlicher Quelle, wie Bodenproben, Biomasseproben und dergleichen gewonnen. Insbesondere stammt die Dehydrogenase aus einer Hefe oder einem Milchsäurebakterium.

Die Dehydrogenase kann in gereinigter oder teilweise gereinigter Form oder in Form des Mikroorganismus selbst verwendet werden. Verfahren zur Gewinnung und Aufreinigung von Dehydrogenasen aus Mikroorganismen sind dem Fachmann hinreichend bekannt, z.B. aus K. Nakamura & T. Matsuda, "Reduction of Ketones" in K. Drauz und H. Waldmann, Enzyme Catalysis in Organic Synthesis 2002, Vol.III, 991-1032, Wiley-VCH, Weinheim. Rekombinante

Verfahren zur Erzeugung von Dehydrogenasen sind ebenfalls bekannt, beispielsweise aus W. Hummel, K. Abokitse, K. Drauz, C. Rollmann und H. Gröger, Adv. Synth. Catal. 2003, 345, Nr. 1 + 2, S. 153-159.

Geeignete Bakterien sind beispielsweise solche der Gattung Pseudomonas, Burkholderia, Agrobacterium, Rhodococcus, Lactobacillus oder Lactococcus. Geeignete Hefen sind beispielsweise solche der Gattung Geotrichum, Pichia, Candida, Hansenula oder Saccharomyces.

Besonders bevorzugt werden Dehydrogenasen aus Hefen und Milchsäurebakterien eingesetzt. Hierunter bevorzugt sind Dehydrogenasen, die aus Hefen der Gattung Geotrichum oder Candida oder die aus Milchsäurebakterien der Gattung Lactobacillus oder Lactococcus gewonnen werden.

Beispiele für Lactobacillus-Arten sind L. brevis, L. kefir, L. plantarum, L. casei, L. acidophilus, L. delbrueckii und L. sanfrancisco.

Beispiele für Candida-Arten sind C. magnoliae, C. rugosa, C. utilis, C. boidinii, C. parapsilosis und C. antarctica.

Beispiele für Geotrichum-Arten sind G. candidum, G. clavatum und G. fermentans.

Besonders bevorzugt verwendet man Dehydrogenasen aus Candida magnoliae, Geotrichum candidum oder Lactobacillus brevis.

Üblicherweise erfolgt die Reduktion mit der Dehydrogenase in Gegenwart eines geeigneten Coenzyms (auch als Cofaktor bezeichnet). Als Coenzym für die Reduktion des Ketons dient üblicherweise NADH und/oder NADPH. Daneben können Dehydrogenasen als zelluläre Systeme eingesetzt werden, die inherent Cofaktor enthalten, oder alternative Redoxmediatoren zugesetzt werden (A. Schmidt, F. Hollmann und B. Bühler "Oxidation of Alcohols" in K. Drauz und H. Waldmann, Enzyme Catalysis in Organic Synthesis 2002, Vol.III, 991-1032, Wiley-VCH, Weinheim).

Üblicherweise erfolgt die Reduktion mit der Dehydrogenase außerdem in Gegenwart eines geeigneten Cosubstrats, welches in der Regel als Reduktionsmittel für das im Verlauf der Reduktion oxidierte Coenzym fungiert und diese somit regeneriert. Beispiele für geeignete Cosubstrate sind Zucker, insbesondere die Hexosen, wie Glucose, Mannose, Fructose, und/oder oxidierbare Alkohole, insbesondere Ethanol, Propanol oder Isopropanol, sowie Formiat. Zur Oxidation des Cosubstrates und damit verbunden zur Regeneration des Coenzyms kann eine zweite Dehydrogenase, wie z.B. Glucose Dehydrogenase bei Verwendung von Glucose als Cosu bstrat, zugesetzt werden. Diese kann als freies oder immobilisiertes Enzym oder in Form von freien oder immobilisierten Zellen eingesetzt werden. Ihre Herstellung kann sowohl separat als auch durch Coexpression in einem (rekombinanten) Dehydrogenase-Stamm erfolgen.

Die erfindungsgemäß verwendeten Dehydrogenasen können frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes" in K. Drauz und H. Waldmann, Enzyme Catalysis in Organic Synthesis 2002, Vol.III, 991-1032, Wiley-VCH, Weinheim beschrieben.

Die Reduktionsreaktion kann in wässrigen oder nichtwässrigen Reaktionsmedien oder in 2-Phasensystemen oder (Mikro-)Emulsionen erfolgen. Bei den wässrigen Reaktionsmedien handelt es sich vorzugsweise um gepufferte Lösungen, die in der Regel einen pH-Wert von 5 bis 8, vorzugsweise von 6 bis 8, aufweisen. Das wässrige Lösungsmittel kann neben Wasser außerdem wenigstens einen Alkohol, z.B. Ethanol oder Isopropanol enthalten.

Unter nicht-wässrigen Reaktionsmedien sollen Reaktionsmedien verstanden werden, die weniger als 1 Gew.-%, vorzugsweise weniger als 0,5 Gew.-% Wasser, bezogen auf das Gesamtgewicht des Reaktionsmediums, enthalten. Vorzugsweise wird die Umsetzung in einem organischen Lösungsmittel durchgeführt. Geeignete Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit einem oder zwei Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether, vorzugsweise mit 4 bis 8 Kohlenstoffatomen, wie Diethylether, Methyl-tert-butylether, Ethyl-tert-butylether, Dipropylether, Diisopropylether, Dibutylether, Tetrahydrofuran oder Ester wie Ethylacetat oder n-Butylacetat oder Ketone wie Methylisobutylketon oder Dioxan oder Gemische davon. Besonders bevorzugt werden die vorgenannten Ether, insbesondere Tetrahydrofuran, verwendet.

Beispielsweise wird die Reduktion mit der Dehydrogenase in einem wässrigorganischen, insbesondere wässrigen Reaktionsmedium durchgeführt.

Das Halogenpropanon III wird vorzugsweise in einer Konzentration von 0,1 g/l bis 500 g/l, besonders bevorzugt von 1 g/l bis 50 g/l in die enzymatische Reduktion eingesetzt und kann kontinuierlich oder diskontinuierlich nachgeführt werden.

Die enzymatische Reduktion erfolgt in der Regel bei einer Reaktionstemperatur unterhalb der Desaktivierungstemperatur der eingesetzten Dehydrogenase und vorzugsweise bei wenigstens -10 °C. Besonders bevorzugt liegt sie im Bereich von 0 bis 100 °C, insbesondere von 15 bis 60 °C und speziell von 20 bis 40 °C, z.B. bei etwa 30 °C.

Zur Durchführung kann man beispielsweise das Halogenpropanon III mit der Dehydrogenase, dem Lösungsmittel und gegebenenfalls den Coenzymen, gegebenenfalls einer Hilfs-Dehydrogenase zur Regenerierung des Coenzyms und/oder weiteren Cosubstraten vorlegen und das Gemisch durchmischen, z. B. durch Rühren oder Schütteln. Es ist aber auch möglich, die Dehydrogenase(n) in einem Reaktor, beispielsweise in einer Säule, zu immobilisieren, und durch den Reaktor eine das Halogenpropanon III und gegebenenfalls Coenzyme und/oder Cosubstrate enthaltende Mischung zu leiten. Hierzu kann man die Mischung im Kreislauf durch den Reaktor leiten bis der gewünschte Umsatz erreicht ist. Dabei wird die Ketogruppe des Halogenpropanons III zu einer OH-Gruppe reduziert, wobei im Wesentlichen selektiv das S-Enantiomer des Halogenpropanols IV-S entsteht. In der Regel wird man die Reduktion bis zu einem Umsatz von wenigstens 70 %, besonders bevorzugt von wenigstens 85 % und insbesondere von wenigstens 95%, bezogen auf das in der Mischung enthaltene Halogenpropanon III führen. Das Fortschreiten der Reaktion, d. h. die sequentielle Reduktion des Ketons, kann dabei durch übliche Methoden wie Gaschromatographie verfolgt werden.

Besonders bevorzugt werden als Dehydrogenasen im erfindungsgemäßen Verfahren Alkohol-Dehydrogenasen mit wenigstens einer der folgenden Eigenschaften eingesetzt:

Alkohol Dehydrogenasen, mit einer Aminosäuresequenz, die im Bereich des N-Terminus a) eine Aminosäureteilsequenz von wenigstens 5, 6, 7 oder 8, vorzugsweise wenigstens 10, wie z.B. 10, 11, 12, 13, 14, oder 15, aufeinanderfolgenden *Aminosäureresten* gemäß SEQ ID NO: 1 umfasst, wobei vorzugsweise zusätzlich die der Aminosäureposition 12 gemäß SEQ ID NO:1 entsprechende Position für Valin steht; oder b) eine Aminosäureteilsequenz von wenigstens 5, 6, 7 oder 8, vorzugsweise wenigstens 10, wie z.B. 10, 11, 12, 13, 14, oder 15, aufeinanderfolgenden *Aminosäureresten* gemäß SEQ ID NO: 2 umfasst; und funktionale Äquivalente davon.

Alkohol Dehydrogenasen weiche zur Reduktion von 3-Chlor-1-(thien-2-yl)-propan-1-on zu (S)-3-Chlor-1-(thien-2-y)-propan-1-ol befähigt ist.

Alkohol Dehydrogenasen, welche die Reduktion in einer Enantiomerenreinheit von wenigstens wenigstens 85 % ee (in Gegenwart von NADH und/oder NADPH; bei 30°C und pH 6.0) katalysiert.

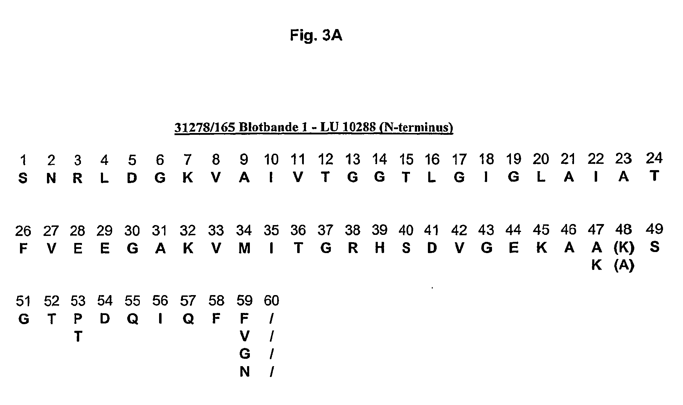

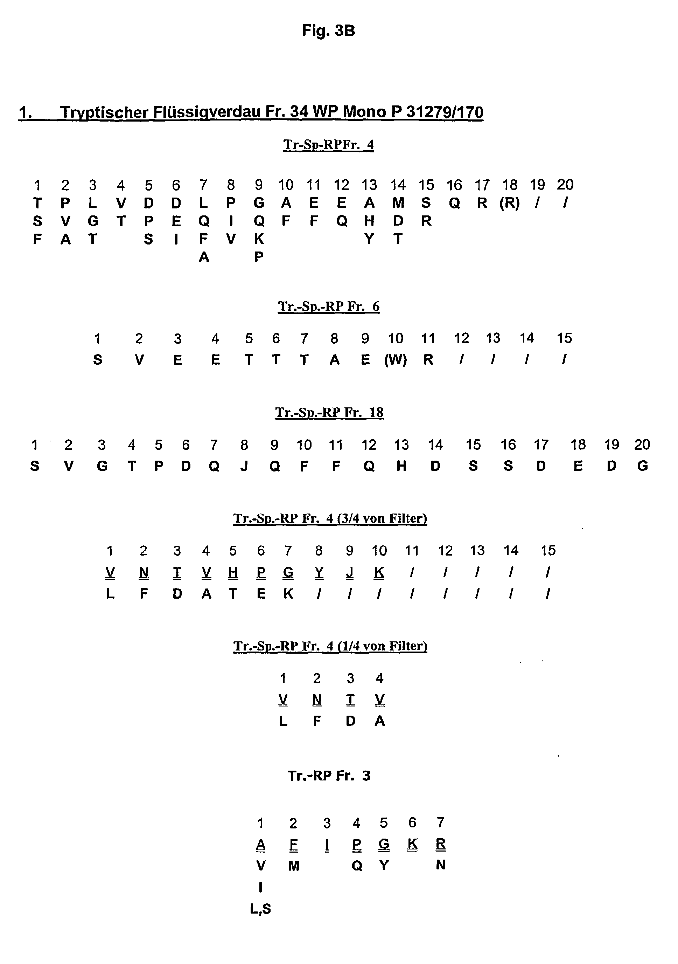

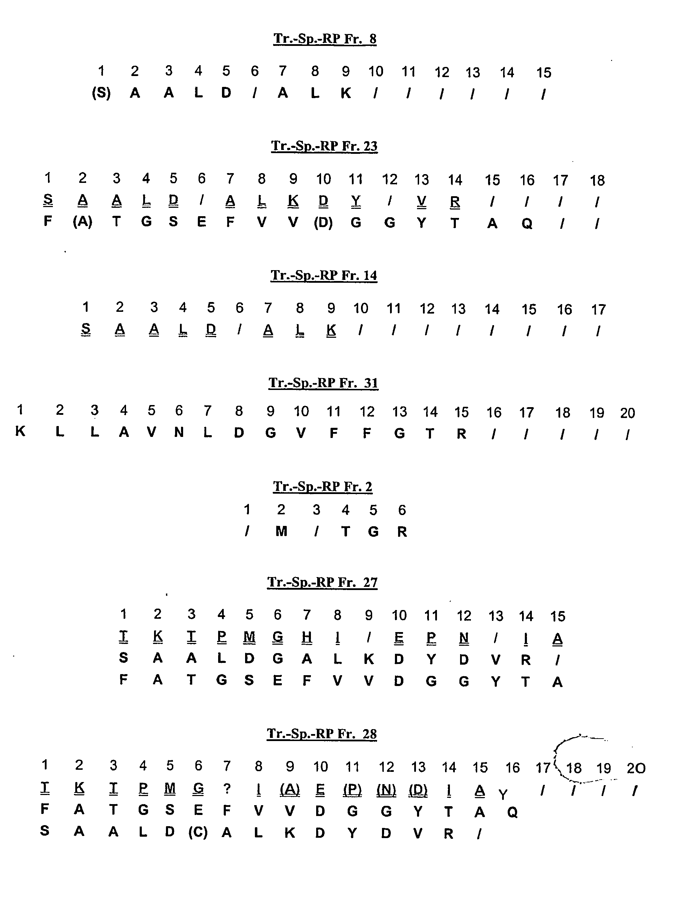

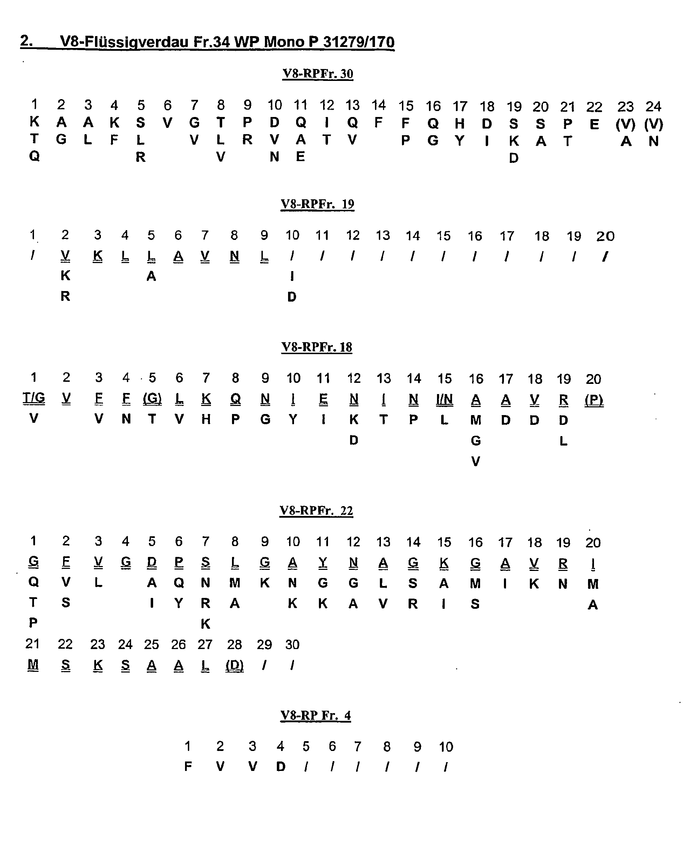

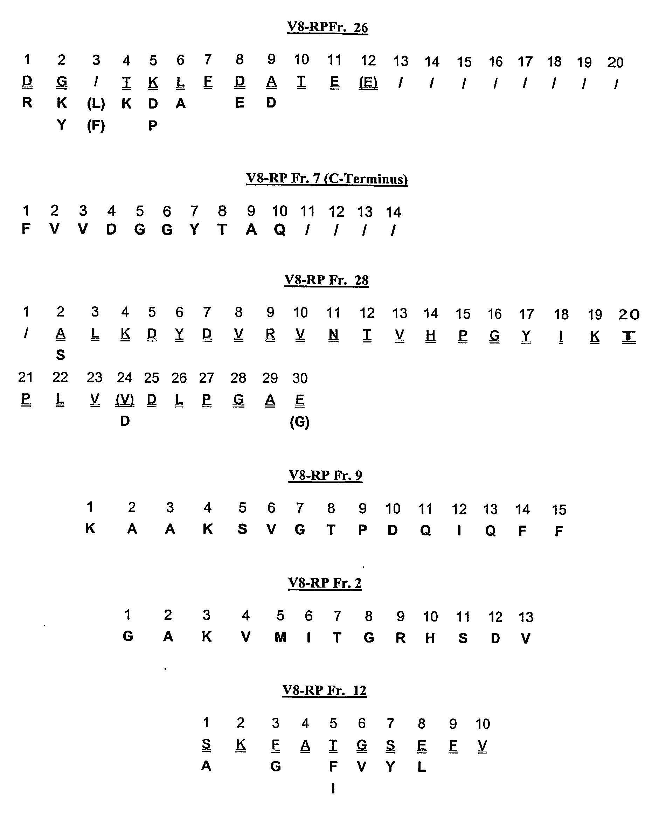

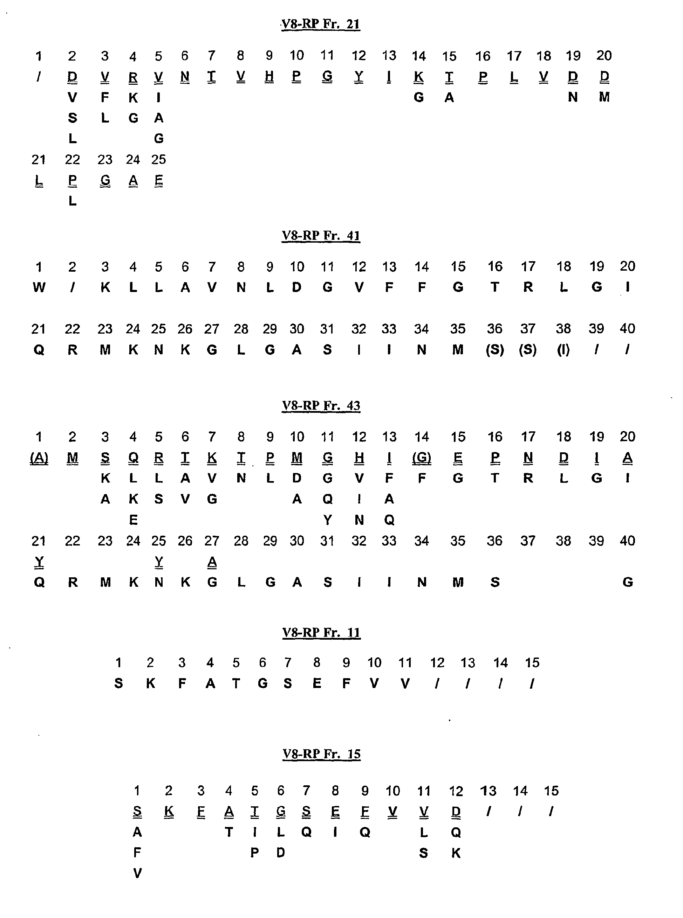

Alkohol Dehydrogenasen, welche von einer Nukleinsäuresequenz, umfassend SEQ ID NO:3, kodiert werden, oder welche eine Aminosäuresequenz gemäß SEQ ID NO: 4 oder wenigstens eine Teilsequenz gemäß Figur 3 umfassen, und vorzugsweise aus Lactobacillus brevis erhältlich sind; sowie die davon abgeleiteten funktional äquivalenten Alkohol Dehydrogenasen.

Alkohol Dehydrogenasen, welche von einer Nukleinsäuresequenz, umfassend SEQ ID NO:5, kodiert werden, oder welche eine Aminosäuresequenz, umfassend SEQ ID NO: 6, aufweisen, und vorzugsweise aus Candida magnoliae (ATCC 12573) erhältlich sind; sowie die davon abgeleiteten funktional äquivalenten Alkohol Dehydrogenasen.

Beschrieben wird auch eine (S)-3-Chlor-1-(thien-2-yl)-propan-1-ol-Dehydrogenase mit wenigstens einer der vorstehend aufgeführten Eigenschaften.

Vorzugsweise liegt der Temperatur-Stabilitätsbereich der Dehydrogenase bei 10 bis 80 °C. Das Temperatur-Optimum ihrer Aktivität liegt vorzugsweise im Bereich von 20 bis 60 °C, besonders bevorzugt von 25 bis 40 °C. Der Stabilitätsbereich für den pH liegt vorzugsweise bei pH 4 bis 10 . Der optimale pH für die Dehydrogenierung liegt vorzugsweise im Bereich von pH 5 bis 8 .

Des weiteren ist die beschriebene Dehydrogenase vorzugsweise zur Dehydrogenierung von (S)-3-Chlor-1-(thien-2-yl)-propan-1-ol zu 3-Chlor-1-(thien-2-yl)-propan-1-on in Gegenwart von $NAD^+$ oder $NADP^+$ befähigt. Bevorzugt weist sie ein Molekulargewicht im Bereich von 30 $\pm$ 2 kDalton auf.

Beschrieben wird außerdem eine Nukleinsäuresequenz, umfassend die kodierende Sequenz für die erfindungsgemäße Dehydrogenase oder eine kodierende Teilsequenz. Ein nichtlimitierendes Beispiel dafür ist die in SEQ ID NO: 3 oder 6 dargestellte Seqeuenz.

Ferner wird beschrieben eine Expressionskassette, umfassend in operativer Verknüpfung mit wenigstens einer regulativen Nukleinsäuresequenz die erfindungsgemäße Nukleinsäuresequenz.

Des weiteren wird beschrieben ein rekombinanter Vektor, umfassend wenigstens eine erfindungsgemäße Expressionskassette.

Außerdem wird beschrieben ein prokaryontischer oder eukaryontischer Wirt, transformiert mit wenigstens einem erfindungsgemäßen Vektor.

Schließlich wird beschrieben die Verwendung der erfindungsgemäßen Dehydrogenase oder eines diese Dehydrogenase produzierenden Mikroorganismus zur Herstellung von (S)-3-Halogen-1-(thien-2-yl)-propan-1-ol und insbesondere zur Herstellung von Duloxetine.

Weitere Abwandlungen der beschriebenen Dehydrogenasen:

Beschrieben sind ebenfalls "funktionale Äquivalente" der konkret offenbarten Enzyme mit (S)-3-Chlor-1-(thien-2-yl)-propan-1-ol-Dehydrogenase - Aktivität und die Verwendung dieser in den erfindungsgemäßen Verfahren.

"Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. Substratspezifität, besitzen. So versteht man beispielsweise unter "funktionalen Äquivalenten" Enzyme, die von 3-Chlor-1-(thien-2-yl)-propan-1-on zum entsprechenden S-Alkohol reduzieren und die mindestens 20 %, bevorzugt 50 %, besonders bevorzugt 75 %, ganz besonders bevorzugt 90 % der Aktivität eines Enzyms, umfassend eine der unter SEQ ID NO:1, 2, 4 oder 6 aufgeführten oder in Figur 3 dargestellten Aminosäuresequenzen, aufweist. Funktionale Äquivalente sind außerdem vorzugsweise zwischen pH 4 bis 10 stabil und besitzen vorteilhaft ein pH-Optimum zwischen pH 5 und 8 sowie ein Temperaturoptimum im Bereich von 20°C bis 80°C.

Unter "funktionalen Äquivalenten" versteht man erfindungsgemäß insbesondere auch Mutanten, welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

Beispiele für geeignete Aminosäuresubstitutionen sind folgender Tabelle zu entnehmen:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktiviät.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure

und Salze mit organischen Säuren, wie Essigsäure und Oxalsäure sind ebenfalls Gegenstand der Erfindung.

"Funktionale Derivate" beschriebener Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

Im Falle einer möglichen Proteinglykosylierung umfassen "funktionale Äquivalente" Proteine des oben bezeichneten Typs in deglykosylierter bzw. glykosylierter Form sowie durch Veränderung des Glykosylierungsmusters erhältliche abgewandelte Formen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der beschriebenen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelte Beeinträchtigung der Fusionsproteinteile) aufweisen. Nichtlimitierende Beispiele für derartige heterologe Sequenzen sind z.B. Signalpeptide oder Enzyme.

"Funktionale Äquivalente" sind Homologe zu den konkret offenbarten Proteinen. Diese besitzen wenigstens 60 %, vorzugsweise wenigstens 75% ins besondere wenigsten 85 %, wie z.B. 90%, 95%, 97% oder 99%, Homologie zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie eines beschriebenen homologen Polypeptids bedeutet insbesondere prozentuale Identität der Aminosäurereste bezogen auf die Gesamtlänge einer der hierin konkret beschriebenen Aminosäuresequenzen.

Homologe der beschriebenen Proteine oder Polypeptide können durch Mutagenese erzeugt werden, z.B. durch Punktmutation oder Verkürzung des Proteins.

Homologe der beschriebenen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

<u>Weitere Ausgestaltung der beschriebenen kodierenden Nukleinsäuresequenzen</u>

Beschrieben sind insbesondere Nukleinsäuresequenzen (einzel- und doppelsträngige DNA- und RNA-Sequenzen, wie z.B. cDNA und mRNA), die für ein Enzym mit beschriebener Dehydrogenase-Aktivität kodieren. Bevorzugt sind Nukleinsäuresequenzen, welche z.B. für Aminosäuresequenzen gemäß SEQ ID NO: 1, 2, 4, 6 oder Figur 3 oder charakteristische Teilsequenzen davon kodieren, oder Nukleinsäuresequenzen gemäß SEQ ID NO: 3 oder 5 oder charakteristische Teilseq uenzen davon umfassen.

Alle hierin erwähnten Nukleinsäuresequenzen sind in an sich bekannter Weise durch chemische Synthese aus den Nukleotidbausteinen, wie beispielsweise durch Fragmentkondensation einzelner überlappender, komplementärer

Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Beschrieben sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalenten, welche z.B. unter Verwendung künstlicher Nukleotidanaloga zugänglich sind.

Die Erfindung beschreibt sowohl isolierte Nukleinsäuremoleküle, welche für erfindungsgemäße Polypeptide bzw. Proteine oder biologisch aktive Abschnitte davon kodieren, als auch Nukleinsäurefragmente, die z.B. als Hybridisierungssonden oder Primer zur Identifizierung oder Amplifizierung von erfindungsgemäßer kodierenden Nukleinsäuren verwendet werden können.

Die Nukleinsäuremoleküle können zudem untranslatierte Sequenzen vom 3'- und/oder 5'-Ende des kodierenden Genbereichs enthalten

Die Erfindung beschreibt weiterhin die zu den konkret beschriebenen Nukleotidsequenzen komplementären Nukleinsäuremoleküle oder einen Abschnitt davon.

Die Nukleotidsequenzen ermöglichen die Erzeugung von Sonden und Primern, die zur Identifizierung und/oder Klonierung von homologer Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa .40, 50 oder 75 aufeinanderfolgende Nukleotide eines Sense-Stranges einer beschriebenen Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein Nukleinsäuremolekül kann mittels molekularbiologischer Standard-Techniken und der bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die Oligonukleotide können ferner durch Standard-Syntheseverfahren, z.B. mit einem automatischen DNA-Synthesegerät, hergestellt werden.

Die Nukleinsäuresequenzen lassen sich prinzipiell aus allen Organismen identifizieren und isolieren. Vorteilhaft lassen sich die Nukleinsäuresequenzen oder die Homologen davon, aus Pilzen, Hefen oder Bakterien isolieren. Als Bakterien seien gram-negative und gram-positive Bakterien genannt. Bevorzugt werden die Nukleinsäuren aus gram-negativen Bakterien vorteilhaft aus alpha-Proteobacterien, beta-Proteobacterien oder gamma-Proteobacterien, besonders bevorzugt aus Bakterien der Familien Enterobacteriaceae, Pseudomonadaceae oder Rhizobiaceae, ganz besonders bevorzugt aus Bakterien der Gattung Agrobacterium, Pseudomonas oder Burkholderia über dem Fachmann bekannte Methoden isoliert.

Nukleinsäuresequenzen lassen sich beispielsweise mit üblichen Hybridisierungsverfahren oder der PCR-Technik aus anderen Pilzen oder Bakterien, z.B. über genomische oder cDNA-Banken, isolieren. Diese DNA-Sequenzen hybridisieren unter Standardbedingungen mit den beschriebenen Sequenzen. Zur Hybridisierung werden vorteilhaft kurze Oligonukleotide der konservierten Bereiche beispielsweise aus dem aktiven Zentrum, die über Vergleiche mit einer beschriebenen Dehydrogenase in dem Fachmann bekannter Weise ermittelt werden können, verwendet. Es können aber auch längere Fragmente der beschriebenen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca 10 °C niedriger als die von DNA:RNA-Hybriden gleicher Länge.

Unter Standardbedingungen sind beispielsweise je nach Nukleinsäure Temperaturen zwischen 42 und 58 °C in einer wäßrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride

liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Beschrieben sind auch Derivate der konkret offenbarten oder ableitbaren Nukleinsäuresequenzen.

So können weitere Nukleinsäuresequenzen z.B. von SEQ ID NO:3 oder 5 abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Beschrieben sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Beschrieben sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Beschrieben sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten einer beschriebenen Nukleinsäuresequenz sind beispielsweise Allelvarianten zu verstehen, die mindestens 40 % Homologie auf der abgeleiteten Aminosäureebene, bevorzugt mindestens 60 % Homologie, ganz besonders bevorzugt mindestens 80, 85, 90, 93, 95 oder 98 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivaten auch Homologe der beschriebenen Nukleinsäuresequenzen, beispielsweise pilzliche oder bakterielle Homologe, verkürzte Sequenzen, Einzelstrang-DNA oder RNA der kodierenden und nichtkodierenden DNA-Sequenz, zu verstehen. Sie besitzten z.B. auf DNA-Ebene eine Homologie von mindestens 40 %, bevorzugt von mindestens 60 %, besonders bevorzugt von mindestens 70 %, ganz besonders bevorzugt von mindestens 80 % über den gesamten angegebenen ONA-Bereich.

Außerdem sind unter Derivaten beispielsweise Fusionen mit Promotoren zu verstehen. Die Promotoren, die den angegebenen Nukleotidsequenzen vorgeschalten sind, können durch ein oder mehrere Nukleotidaustausche, Insertionen, Inversionen und/oder Deletionen verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Unter Derivaten sind auch Varianten zu verstehen, deren Nukleotidsequenz im Bereich von -1 bis -1000 Basen stromaufwärts vor dem Startkodon oder 0 bis 1000 Basen stromabwärts nach dem Stopkodon so verändert wurden, dass die Genexpression und/oder die Proteinexpression verändert, bevorzugt erhöht wird.

Weiterhin beschriebt die Erfindung auch Nukleinsäuresequenzen, welchen mit oben genannten kodierenden Sequenzen unter "stringenten Bedingungen" hybridisieren. Unter dieser Eigenschaft versteht man die Fähigkeit eines Poly- oder Oligonukleotids unter stringenten Bedingungen an eine nahezu komplementäre Sequenz zu binden, während unter diesen Bedingungen unspezifische Bindungen zwischen nichtkomplementären Partnern unterbleiben. Dazu sollten die Sequenzen zu 70-100%, vorzugsweise zu 90-100%, komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze. Üblicherweise werden dazu Oligonukleotide ab einer Länge von 30 Basenpaaren eingesetzt. Unter stringenten Bedingungen versteht man beispielsweise in der Northern-Blot-Technik die Verwendung einer 50 - 70 °C, vorzugsweise 60 - 65 °C warmen Waschlösung, beispielsweise 0,1 x SSC-Puffer mit 0, 1 % SDS (20x SSC: 3M NaCl, 0,3M Na-Citrat, pH 7,0) zur Elution unspezifisch hybridisierter cDNA-Sonden oder Oligonukleotide. Dabei bleiben, wie oben erwähnt, nur in hohem Maße komplementäre Nukleinsäuren aneinander gebunden. Die Einstellung stringenter Bedingungen ist dem Fachmann bekannt und ist z:B. in Ausubel et al., Current Protocols in Molecular Biology, John Wiley & Sons, N.Y. (1989), 6.3.1-6.3.6. beschrieben.

Diese Polynukleotide lassen sich bei der Durchmusterung von genomischen oder cDNA-Banken auffinden und gegebenenfalls daraus mit geeigneten Primern mittels PCR vermehren und anschließend beispielsweise mit geeigneten

Sonden isolieren. Darüber hinaus können Polynukleotide auch auf chemischem Wege synthetisiert werden.

Ausgestaltungen der Konstrukte

Beschrieben sind außerdem Expressionskonstrukte, enthaltend unter der genetischen Kontrolle regulativer Nukleinsäuresequenzen eine für ein erfindungsgemäßes Polypeptid kodierende Nukleinsäuresequenz; sowie Vektoren, umfassend wenigstens eines dieser Expressionskonstrukte.

Vorzugsweise umfassen solche beschriebenen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Enhancer, Polyadenylierungssignale und dergleichen. Weitere regulative Elemente umfassen selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Unter einem beschriebenen Nukleinsäurekonstrukt sind insbesondere solche zu verstehen, bei weichen das Gen für eine beschriebene Dehydrogenase mit einem oder mehreren Regulationssignalen zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die. Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die erfindungsgemäßen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Vorteilhafte Regulationssequenzen für das erfindungsgemäße Verfahren sind beispielsweise in Promotoren wie cos-, tac-, trp-, tet-, trp-tet-, lpp-, lac-, lpp-lac-, $lacI^{q-}$, T7-T5-, T3-, gal-, trc-, ara-, rhaP ($rhaP_{BAD}$)SP6-, lambda-$P_R$- oder im lambda-$P_L$-Promotor enthalten, die vorteilhafterweise in gram-negativen Bakterien Anwendung finden. Weitere vorteilhafte Regulationssequenzen sind beispielsweise in den grampositiven Promotoren amy und SPO2, in den Hefe- oder Pilzpromotoren ADC1, MFalpha, AC, P-60, CYC1, GAPDH, TEF, rp28, ADH enthalten. In diesem Zusammenhang sind auch die Promotoren der Pyruvatdecarboxylase und der Methanoloxidase, beispielsweiseaus Hansenula vorteilhaft. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar. Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, pIN-$III^{113}$-B1, λgt11 oder pBdCl, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, $pGHlac^+$, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985, ISBN 0 444 904018) entnommen werden.

Vorteilhafterweise enthält das Nukleinsäurekonstrukt zur Expression der weiteren enthaltenen Gene zusätzlich noch 3'- und/oder 5'-terminale regulatorische Sequenzen zur Steigerung der Expression, die je nach ausgewähltem Wirtorganismus und Gen oder Gene für eine optimale Expression ausgewählt werden.

Diese regulatorischen Sequenzen sollen die gezielte Expression der Gene und der Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

In einer weiteren Ausgestaltungsform des Vektors kann der das Nukleinsäurekonstrukt oder die Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäßen Nukleinsäure bestehen.

Für eine optimale Expression heterologer Gene in Organismen ist es vorteilhaft die Nukleinsäuresequenzen entsprechend des im Organismus verwendeten spezifischen "codon usage" zu verändern. Der "codon usage" läßt sich anhand von Computerauswertungen anderer, bekannter Gene des betreffenden Organismus leicht ermitteln.

Die Herstellung einer Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

Brauchbare Wirtsorganismen

Mit Hilfe der beschriebenen Vektoren oder Konstrukte sind rekombinante Mikroorganismen herstellbar, welche beispielsweise mit wenigstens einem erfindungsgemäßen Vektor transformiert sind und zur Produktion der erfindungsgemäßen Polypeptide eingesetzt werden können. Vorteilhafterweise werden die oben beschriebenen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Auch homolog rekombinierte Mikroorganismen sind herstellbar. Dazu wird ein Vektor hergestellt, der zumindest einen Abschnitt eines erfindungsgemäßen Gens oder einer kodierenden Sequenz enthält, worin gegebenenfalls wenigstens eine Aminosäure-Deletion, -Addition oder -Substitution eingebracht worden ist, um die beschriebene Sequenz zu verändern, z.B. funktionell zu disrumpieren ("Knock-out"-Vektor). Die eingebrachte Sequenz kann z.B. auch ein Homologes aus einem verwandten Mikroorganismus sein oder aus einer Säugetier-, Hefe- oder Insektenquelle abgeleitet sein. Der zur homologen Rekombination verwendete Vektor kann alternativ derart ausgestaltet sein, daß das endogene Gen bei homologer Rekombination mutiert oder anderweitig verändert ist, jedoch noch das funktionelle Protein kodiert (z.B. kann der stromaufwärts gelegene regulatorische Bereich derart verändert sein, dass dadurch die Expression des endogenen Proteins verändert wird). Der veränderte Abschnitt des erfindungsgemäßen Gens ist im homologen Rekombinationsvektor. Die Konstruktion geeigneter Vektoren zur homologen Rekombination ist z.B. beschrieben in Thomas, K.R. und Capecchi, M.R. (1987) Cell 51:503.

Als rekombinante Wirtsorganismen für die beschriebene Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden.

Der Wirtsorganismus oder die Wirtsorganismen enthalten dabei vorzugsweise mindestens eine der in dieser Erfindung beschriebenen Nukleinsäuresequenzen, Nukleinsäurekonstrukte oder Vektoren, die für ein Enzym mit erfindungsgemäßer Dehydrogenaseaktivität kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu beginn der Fermentation vorgelegt oder semikontinuierlich oder kontinuierlich nachgefüttert werden. Das Keton kann direkt zur Anzucht gegeben werden oder vorteilhaft nach Anzucht. Die Enzyme können nach dem in den Beispielen beschriebenen Verfahren aus den Organismen isoliert werden oder als Rohextrakt für die Reaktion verwendet werden.

Rekombinante Herstellung der beschriebenen Polypeptide

Beschrieben sind weiterhin Verfahren zur rekombinanten Herstellung erfindungsgemäße Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Der rekombinante Mikroorganismus kann nach bekannten Verfahren kultiviert und fermentiert werden. Bakterien können beispielsweise in TB- oder LB-Medium und bei einer Temperatur von 20 bis 40°C und einem pH-Wert von 6 bis 9 vermehrt werden. Im Einzelnen werden geeignete Kultivierungsbedingungen beispielsweise in T. Maniatis, E.F. Fritsch and J. Sambrook, Molecular Cloning: A Laboratory Manual, Cold Spring Harbor Laboratory, Cold Spring Harbor, NY (1989) beschrieben.

Die Zellen werden dann, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, Ionenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, die beispielsweise in einer Chromatographiesäule eingefüllt sein kann, oder an einer Mikrotiterplatte oder an einem sonstigen Träger dienen.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

Weitere Ausgestaltungen zur Durchführung des erfindungsgemäßen enzymatischen Reduktionsverfahrens

Die Dehydrogenasen können im erfindungsgemäßen Verfahren als freies oder immobilisiertes Enzym verwendet werden.

Das erfindungsgemäße Verfahren wird vorteilhaft bei einer Temperatur zwischen 0 °C bis 95 °C, bevorzugt zwischen 10 °C bis 85 °C, besonders bevorzugt zwischen 15 °C bis 75 °C durchgeführt.

Der pH-Wert im erfindungsgemäßen Verfahren wird vorteilhaft zwischen pH 4 und 12, bevorzugt zwischen pH 4,5 und 9, besonders bevorzugt zwischen pH 5 und 8 gehalten.

Unter enantiomerenreinen bzw. chiralen Produkten, wie 3-Chlor-1-(thien-2-yl)-(S)-propan-1-ol, sind im erfindungsgemäßen Verfahren Enantiomere zu verstehen, die eine Enantiomerenanreicherung zeigen. Bevorzugt werden

im Verfahren Enantiomerenreinheiten von mindestens 70 %ee, bevorzugt von min. 80 %ee, besonders bevorzugt von min. 90 %ee, ganz besonders bevorzugt min. 98 %ee erreicht.

Für das erfindungsgemäße Verfahren können wachsende Zellen verwendet werden, die die erfindungsgemäßen Nukleinsäuren, Nukleinsäurekonstrukte oder Vektoren enthalten. Auch ruhende oder aufgeschlossene Zellen können verwendet werden. Unter aufgeschlossenen Zellen sind beispielsweise Zellen zu verstehen, die über eine Behandlung mit beispielsweise Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder angereinigte Enzyme können für das Verfahren verwendet werden. Ebenfalls geeignet sind immobilisierte Mikroorganismen oder Enzyme, die vorteilhaft in der Reaktion Anwendung finden können.

Das erfindungsgemäße Verfahren kann batchweise, semi-batchweise oder kontinuierlich betrieben werden.

Die Durchführung des Verfahrens kann vorteilhafterweise in Bioreaktoren erfolgen, wie z.B. beschrieben in Biotechnology, Band 3, 2. Auflage, Rehm et al Hrsg., (1993) insbesondere Kapitel II.

Die nachfolgenden Beispiele sollen die Erfindung veranschaulichen, ohne sie jedoch einzuschränken. Hierbei wird auf beiliegende Figuren Bezug genommen, dabei zeigt:

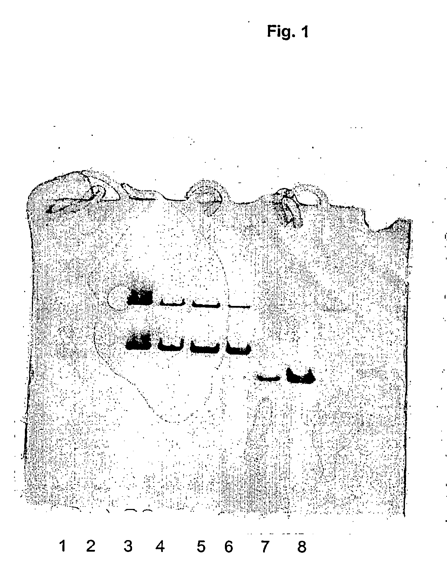

Figur1 das aktivitätsgefärbte Gel einer erfindungsgemäß isolierten Dehydrogenase aus Lu10288; Spur 1: Molekulargewichtsstandards von unten: 47 kDa, 74 kDa, 121 kDa, 205 kDa; Spur 2: leer; Spur 3: Homogenat Überstand; Spur 4: Wertfraktion Q-Sepharose; Spur 5: Wertfraktion Q-Sepharose (dreifache Menge); Spur 6: Wertfraktion Superdex; Spur 7: Wertfraktion Mono-Q; Spur 8: Wertfraktion Mono-P;

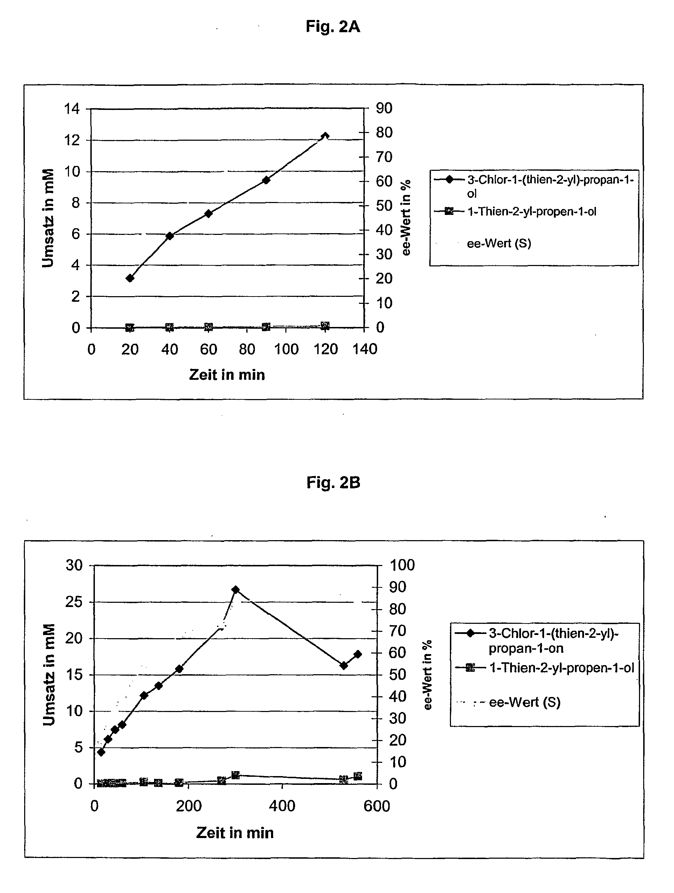

Figur 2A und 2B einen typische Reaktionsverlauf verschiedener Ansätze zur Herstellung von (S)-3-Methylamino-1-(thien-2-yl)-propan-1-ol I-S durch Reduktion mit einer Dehydrogenase aus Lactobacillus brevis.

Figur 3A das Ergebnis der N-terminalen Sequenzierung einer Blotbande von erfindungsgemäß isolierter Dehydrogenase aus Lu10288; und Figur 3B die Sequenzierdaten verschiedener proteolytischer Fragmente einer erfindungsgemäß gereinigten Dehydrogenase aus Lu10288; sofern bei mehreren Signalen pro Sequenzierschritt eine Hauptsequenz feststellbar war, ist diese doppelt unterstrichen dargestellt. Nicht lesbare Positionen sind mit"/" gekennzeichnet; für Positionen deren Bestimmung mit einer gewissen Unsicherheit behaftet ist, wird der Aminosäurerest in Klammern angegeben. Zwei Aminosäuren sind in einer Position angegeben, wenn keine eindeutige Präferenz angegeben weden kann. "?" bedeutet eine unbekannte, bzw. nicht bestimmbare Aminosäure.

## Beispiele

### A. Chemische Beispiele

**Beispiel 1:** Herstellung von 3-Chlor-1-(thien-2-yl)-propan-1-on III.1

33 kg Thiophen wurden in 150 kg Dichloethan vorgelegt und anschließend mit 62,7 kg Aluminiumtrichlorid versetzt. Das Reaktionsgemisch wurde auf 10 °C gekühlt und mit 55 kg 3-Chlorpropionsäurechlorid versetzt. Das Gemisch wurde anschließend 12 h bei Raumtemperatur gerührt. Die GC- und NMR-Kontrolle zeigten, dass das Reaktionsgemisch noch etwa 3-4 Moi-% 3-Chlor-1-(thien-2-yl)-propenon, bezogen auf die Summe aus gebildetem 3-Chlor-1-(thien-2-yl)-propan-1-on und 3-Chlor-1-(thien-2-yl)-propenon, enthielt. Daraufhin leitet man bei Raumtemperatur so lange (ca. 30 Minuten) Chlorwasserstoff ein, bis kein Propenon mehr nachweisbar war. Anschließend hydrolysierte man das Reaktionsgemisch durch Zugabe von 100 kg entionisiertem Wasser. Die organische Phase wurde abgetrennt und mit 100 kg entionisiertem Wasser gewaschen. Nach Entfernen des Lösungsmittels im Vakuum erhielt man 65,7 kg (96 % der Theorie) der Titelverbindung als Öl.

**Beispiel 2:** Herstellung von 3-Methylamino-1-(thien-2-yl)-propan-1-ol I durch Reduktion mit Natriumborhydrid

Das Propanon aus Beispiel 1 wurde in einem Gemisch aus 400 ml Toluol und 200 g Methanol bei 0 °C vorgelegt. Nach Zugabe von 2 g 30%iger wässriger Natriumhydroxidlösung wurden innerhalb von 2,5 h 21,4 g Natriumborhydrid portionsweise zugegeben. Nach 40 min Rühren bei 0 °C wurde das Reaktionsgemisch mit 12,1 g 40%ige wässrige Metylaminlösung versetzt. Das Gemisch wurde 6 h bei 60 °C unter Eigendruck gerührt. Nach dem Abkühlen auf Raumtemperatur entfernte man das Lösungsmittel, digerierte den Rückstand mit Toluol und filtrierte davon ab. Das Filtrat wurde getrocknet und man erhielt 175,8 g der Titelverbindung in Form eines hellgelben Feststoffs.

**B. Biochemische Beispiele:**

**Beispiel 3:** Bereitstellung von Glucose-Dehydrogenase zur Cofaktor-Regenerieru ng

Zur Cofaktor-Regenerierung konnte Glucose-Dehydrogenase aus kommerziellen Quellen (z.B. Jülich Fine Chemicals Order-No. 22.10 oder 19.10) oder eigenen Quellen verwendet werden, wobei letztere ein E.coli XL10 Gold pUC19-Klon war, der das Glucose-Dehydrogenase-Gen aus Bacillus subtilis (Genbank-Acc.No. M12276) enthielt (Lu11293).
Zur Fermentation von E. coli Lu11293 wurde folgendes Medium angesetzt:

| | |
|---|---|
| 560 g | Hefeextrakt (65 %) |
| 448 g | Trypton (Difco) |
| 42 g | $KH_2PO_4$ |
| 84 g | $Na_2HPO_4$ |
| 644 g | Glycerin (99%) |
| 100 mL | SL4 Lösung (5 fach) |
| | |
| 1 g | Tegosipon 3062 |
| | Medium mit Wasser auf 13,5 L auffüllen, pH-Wert |
| | auf 7,0 einstellen, ca. 300 mL für Vorkultur ent- |
| | nehmen, danach 30 min. bei 122 °C sterilisieren. |
| | |
| | Sterile Salzlösung* zugeben (vorher die Salzlösung für |
| | die Schüttelkolben entnehmen, siehe Rapport). |

| | | |
|---|---|---|
| *Salzlösung: | 2,1 g | $CaCl_2 * 2 H_2O$ |
| | 3,5 g | $MgSO_4 * 7 H_2O$ |
| 14 g | $NH_4Cl$ | |
| + 14 mL | Ampicillin-Lösung (100 mg/mL) | |
| in 500 mL | Wasser lösen und sterilfiltrieren | |

Jeweils 150 ml Medium wurden in zwei 1 l Erlenmeyerkolben sterilisiert und mit 5 ml steriler Salzlösung komplettiert. Nach Beimpfen von einer LB-Ampicillin-Agarplatte wurden die Vorkulturen 12 Stunden bei 37 °C und 200 UPM inkubiert und zum Fermentationsmedium gegeben. Die Fermentation wurde bei 37°C, 0,1 bar Innendruck, pH 7,0 (Regelung mit 20% Phosphorsäure und 25% NaOH) mit einer Begasungsrate von 7,5 L/min und 300 upm gestartet (Regelung $pO_2$ zwischen 20 und 50% mit 10-20 L/min Zuluft und 500-1500 Upm). Nach 2 h wurden zur Induktion 0,1 mM IPTG zugegeben und nach insgesamt 13 h die Fermentation beendet. Nach Ernte und Waschen der Zellen (1,3 kg) wurden diese bis zur Verwendung (2-20 g/L im Ansatz) bei -20°C gelagert.

**Beispiel 4:** Screening nach Dehydrogenasen zur Reduktion von 3-Chlor-1-(thien-2-yl)-propan-1-on

a) Verschiedene Bakterien- und Pilzstämme (überwiegend aus verschiedenen Stammsammlungen) wurden 24 bis 48 h in 20 ml LB-Medium, MRS-Medium (Fa.Difco) oder GYP-Medium (verwendet für Hefen) (1 % w/v D-Glucose, je 0.5 % w/v Hefeextrakt und Polypepton, pH 6.0) bei 30 oder 37°C angezogen, in 3mM Tris-HCl pH7.5 gewaschen und in 2 ml Puffer resuspendiert. Ein Aliquot wurde mit 1 Vol. Glaskugeln (0,3-0,5mm Durchmesser) in der Schwingmühle aufgeschlossen (3x5 min mit Zwischenkühlung á 10 min auf Eis). Nach Abtrennung der Trübung durch Zentrifugation (5 min 14000 U/min 4°C) wurde ein klarer Rohextrakt erhalten. Die Zellsuspensionen und Rohextrakte wurden anschließend getestet auf reduktive Aktivität für 3-Chlor-1-(thien-2-yl)-propan-1-on aus Beispiel 3).

Verwendeter Assay:

| | |
|---|---|
| 150 µl | Zellen/Rohextrakt |
| 50 µl | 1 M Glukoselösung |
| 50 µl | Glukose-Dehydrogenase (12-15 U/µl; 20-200 mg BFM/ml) aus Beispiel 3 |
| 50 µl | 2 mM NADH |
| 50 µl | 2 mM NADPH |
| 10 µl | 0,5 M 3-Chlor-1-(thien-2-yl)-propan-1-on in Methanol |
| 190 µl | 50 mM MES pH 6,0 oder Tris-HCl pH 7,5 |

Die Inkubation erfolgte bei 30 °C. Die Proben (200 µl) wurden mit 3 µl konz HCl nach 1, 2 oder 24 h abgestoppt. Nach Zentrifugation wurden die Überstände per HPLC auf 3-Chlor-1-(thien-2-yl)-propan-1-on und 3-Chlor-1-(thien-2-yl)-propan-1-ol untersucht (Chromolith Speed ROD, RP-18e 50 - 4,6mm, Fluß 1,5 ml/min, 0,0 - 1,0 min: 35% Acetonitril+ 65% 10mM $KH_2PO_4$ Puffer pH 2,5; 1,1 - 1,3 min: 80% Acetonitril+ 20% 10mM $KH_2PO_4$ Puffer pH 2,5; 1,3 - 2,0 min: 35% Acetonitril+ 65% 10mM $KH_2PO_4$ Puffer pH 2,5; Detektion bei 230 nm (Alkohol) und 260 nm (Keton) bei einer Retentionszeit Rt = 1,250 min (3-Chlor-1-(thien-2-yl)-propan-1-ol) und Rt = 1,500 min (3-Chlor-1-(thien-2-yl)-propan-1-on); eventuelle Nebenprodukte aus HCl-Eliminierung (1-Thien-2-yl-propen-1-on und 1-Thien-2-yl-propen-1-ol) bei Rt = 0,971 min und Rt = 1,165 min).

Ausgewählte Stämme wurden wiederholt getestet, wobei zur Bestimmung des Enantiomerenüberschusses (ee) die Proben mit Methyl-tert.-butylether (MTBE) oder Methylisobutylketon (MIBK) extrahiert und per chiraler GC- oder HPLC-Analytik charakterisiert wurden. Folgende Methoden wurden hierzu verwendet: GC: Hydrodex-β-6-TBDM, 25 m, 90°C 10min 5°C/min 180°C 10min, Laufzeit: 38 min, Inlets: Heater: 200°C, Pressure: 106.8 kPa, Total Flow: 102 ml/min, Split Ratio: 125:1, Split Flow: 99.8 ml/min, Detector: 200°C bzw. HPLC: Chiracel OD-H, 250*4,6mm (Daicel), 40°C, Fluß 1,0 ml/min, 0,0 - 1,0 min: 97,5% n-Hexan+2,5% Isopropanol; Detektion bei 230 nm (Alkohol) und 260 nm (Keton) bei Rt 9,50 (3-Chlor-1-(thien-2-yl)-propan-1-on), Rt 16,60 min (R-3-Chlor-1-(thien-2-yl)-propan-1-ol) und Rt 18,30 min (S-3-Chlor-1-(thien-2-yl)-propan-1-ol).

b) Verschiedene Lactobacillus-Stämme wurden neu isoliert

Die Lactobacillus brevis-Stämme Lu10288,10290 und 10291 wurden wie folgt isoliert.

b1) Verwendete Medien

Kleymanns-Medium:

In 915ml werden folgende Substanzen gelöst
10g/l Trypton (Difco-Becton Dicinson)
7g/l Hefe Extrakt (Difco-Becton Dicinson)
2g/l Beef Extrakt (Difco-Becton Dicinson)
5 g/l Fructose
2 g/l Maltose
3,6 g/l Gluconsäure 50%
1,9 g/l Citronensäure*$H_2O$
5 g/l Acetat
1 g/l Tween 80
0,2 g/l $MgSO_4$*$7H_2O$
0,05 g/l $MnSO_4$
0,01 g/l $FeSO_4$
0,4 g/l L-Cystein
1,25 ml $NH_4OH$ 25%
für Agarplatten: Zusatz von 2% Bacto Agar (Difco-Becton Dicinson)
Der pH der Lösung wurde auf 5,4 eingestellt.
Die Lösung wurde 15' bei 121°C autoklaviert.

Nach dem Autoklavieren wurden 50 ml sterile Glucoselösung (5g ad 50 ml $H_2O$ und 40ml Ethanol unter Rühren zugesetzt und Agar-Platten gegossen.

KMB-Medium:

10g/l Trypton (Difco-Becton Dicinson)
7g/l Hefe Extrakt (Difco-Becton Dicinson)

2 g/l $KH_2PO_4$
1 g/l Tween 80
0,5 g/l $MgSO_4*7H_2O$
1 g/l $MnSO_4$
20g/l $CaCO_3$
10g/l Glucose
1,5% Agar für die Herstellung von Agarplatten

Die Lösung wurde 15' bei 121°C autoklaviert.
Die Glucose und das $CaCO_3$ (Riedel de Haen) wurden separatat gelöst und autoklaviert und vor dem Plattengießen mit dem Agar vermischt.
b2) Isolierung der Mikroorganismen:

5-10 g Mais-Silage aus dem Norddeutschen Raum (LUFA-Oldenburg) wurde in einem 50 ml Erlenmeyerkolben anaerob bei 37 °C über Nacht mit 20 ml Saline inkubiert. Der erhaltenen flüssige Anteil wurde 1:100 in 50 ml Kleymanns Medium überimpft und anaerob 24h bei RT und leichtem Rühren inkubiert. Danach wurde die Zellsuspension auf die beschriebenen Kleymanns-Selektionsagarplatten ausplattiert. Die Platten wurden anaerob für 48-72h bei 37°C inkubiert und die erhaltenen Kolonien durch wiederholtes Ausstreichen auf KMB-Medium isoliert. In flüssigem KMB-Medium mit 20 g/l Fructose (24 h 37°C, 50 Upm) fand die Charakterisierung der Stämme durch Bestimmung des Säure-Fermentationsmusters statt (pH-Messung und Konzentrationsbestimmung von Glucose, Fructose, Lactat, Acetat, Ethanol per HPLC-Analyse der Kulturüberstände). Heterofermentative Stämme mit Acetat- und Lactatbildung wurden isoliert und per Analyse der 16sRNA systematisch charakterisiert.

c) Screening-Ergebnisse
Die Tabellen 1,2 und 3 zeigen beispielhafte Stämme bzw. die Umsätze und ee-Werte.

Tabelle 1

| Lu Nr.: | Gattung | Art | Sammlung | Nummer |
|---|---|---|---|---|
| 44 | Byssochlamys | fulva | IMI | 163641 |
| 105 | Geotrichum | candidum | ATCC | 28747 |
| 106 | Geotrichum | candidum | ATCC | 20141 |
| 582 | Pichia | glucozyma | ATCC | 18938 |
| 716 | Hansenula | polymorpha | - | |
| 908 | Saccharomyces | rouxi | IFO | 493 |
| 1844 | Kluyveromyces | lactis | ATCC | 56498 |
| 2707 | Saccharomyces | cerevisiae | ATCC | 9080 |
| 3458 | Geotrichum | candidum | ATCC | 34614 |
| 896 | Geotrichum | vanrij | ATCC | 22375 |
| 897 | Geotrichum | fermentans | ATCC | 56301 |
| 3458 | Geotrichum | klebahnii | ATCC | 20001 |
| 4986 | Candida | utilis | - | |
| 8472 | Candida | magnoliae | ATCC | 12573 |
| 1821 | Candida | guilliermondii | ATCC | 20403 |
| 1823 | Candida | guilliermondii | ATCC | 20474 |
| 8478 | Candida | tropicalis | ATCC | 24887 |
| 8833 | Rhodotorula | aurantiaca | ATCC | 32770 |
| 127 | Pseudomonas | desmolytica | NRRL | 3108 |

(fortgesetzt)

| Lu Nr.: | Gattung | Art | Sammlung | Nummer |
|---|---|---|---|---|
| 404 | Rhodococcus | fragi | IFO | 12049 |
| 444 | Pseudomonas | paucimobilis | ATCC | 10829 |
| 493 | Pseudomonas | citronellolis | ATCC | 13674 |
| 4006 | Pseudomonas | lemoignei | NCIMB | 9947 |
| 8099 | Burkholderia | gladioli | ATCC | 25417 |
| 8510 | Rhodococcus | ruber | DSMZ | 8316 |
| 10288 | Lactobacillus | brevis | * | |
| 10290 | Lactobacillus | brevis | -* | |
| 10291 | Lactobacillus | brevis | -* | |

Tabelle 2 Umsätze:

| Lu Nr.: | Gattung | Art | OD 600 | Umsatz 2h/mM | Umsatz 24h/mM |
|---|---|---|---|---|---|
| 44 | Byssochlamys | fulva | n.b. | 0,0 | 0,2 |
| 105 | Geotrichum | candidum | 12,18 | 0,2 | 0,2 |
| 106 | Geotrichum | candidum | 7,8 | 3,1 | 4,1 |
| 582 | Pichia | glucozyma | 9,98 | 0,1 | 0,1 |
| 716 | Hansenula | polymorpha | 12,24 | 0,0 | 0,3 |
| 908 | Saccharomyces | rouxi | 8,78 | 0,0 | 0,3 |
| 1844 | Kluyveromyces | lactis | 13,86 | 0,0 | 0,3 |
| 2707 | Saccharomyces | cerevisiae | 10 | 0,2 | 0,4 |
| 3458 | Geotrichum | candidum | 8,26 | 0,2 | 0,4 |
| 896 | Geotrichum | vanrij | 11,36 | 0,03 | n.b. |
| 897 | Geotrichum | fermentans | 9,30 | 0,04 | n.b. |
| 3458 | Geotrichum | klebahnii | 10,84 | 0,08 | n.b. |
| 4986 | Candida | utilis | 11,06 | 0,0 | 0,2 |
| 1821 | Candida | guilliermondii | 5,76 | 0,09 | n.b. |
| 1823 | Candida | guilliermondii | 5,54 | 0,07 | n.b. |
| 8472 | Candida | magnoliae | 13,78 | 0,8 | 0,8 |
| 8478 | Candida | tropicalis | 2,6 | 0,05 | n.b. |
| 8833 | Rhodotorula | aurantiaca | 2,32 | 0,1 | 0,3 |
| 127 | Pseudomonas | desmolytica | 3,74 | 0,0 | 0,4 |
| 404 | Rhodococcus | fragi | 1,78 | n.b. | 0,3 |
| 493 | Pseudomonas | citronellolis | 3,54 | n.b. | 0,2 |
| 4006 | Pseudomonas | lemoignei | 2,24 | n.b. | 0,2 |
| 8099 | Burkholderia | gladioli | 15,34 | 0,1 | 0,2 |
| 8510 | Rhodococcus | ruber | 1,1 | 0,0 | 0,1 |
| 10288 | Lactobacillus | brevis | 4,89 | 0,2 | 0,4 |
| 10290 | Lactobacillus | brevis | 2,08 | 0,1 | 0,2 |

(fortgesetzt)

| Lu Nr.: | Gattung | Art | OD 600 | Umsatz 2h/mM | Umsatz 24h/mM |
|---|---|---|---|---|---|
| 10291 | Lactobacillus | brevis | 2,84 | 0,2 | 0,2 |
| n.b.; nicht bestimmt<br>Umsätze bezogen auf gebildeten 3-Chlor-1-(thien-2-yl)-propan-1-ol in mM | | | | | |

Tabelle 3 Testwiederholung mit ee-Wertbestimmung

| Lu Nr.: | Gattung | Art | OD 600 | Umsatz 2h/mM | ee-Wert 24h/% |
|---|---|---|---|---|---|
| 10288 | Lactobacillus | brevis | 4,89 | 5,2 | n.b. |
| 105 | Geotrichum | candidum | 7,86 | 4,2 | 98 |
| 8472 | Candida | magnoliae | 6,56 | 0,13 | 56 |

**Beispiel 5:** Reinigung der Dehydrogenase aus Lactobacillus brevis

Zur Fermentation von Lactobacillus brevis Lu10288 wurde folgendes Medium angesetzt:

| | |
|---|---|
| 1400 g | Hefeextrakt (65 %) |
| 44,1 g | Citronensäure |
| 63 g | $KH_2PO_4$ |
| 21,5 g | $MgSO_4$ * 7 $H_2O$ |
| 4,1 g | $MnSO_4$ * $H_2O$ |
| 21 g | Tween 80 |
| 15,4 g | $(NH_4)_2Fe(SO_4)_2 \cdot 12\ H_2O$ |
| 1 g | Tegosipon 3062 |
| | Medium mit Wasser auf 12,6 l auffüllen, pH-Wert auf 5,8 einstellen, ca. 300 ml für Vorkulturen entnehmen; Sterilisation 30 min. bei 122 °C; 840 g Glukose +860 ml Wasser lösen und sterilisieren; je 15 ml Glukoselösung zu 135 ml Vorkulturmedium in 1L-Kolben geben, Rest zum Fermentationsmedium zufügen. |

2 Vorkulturen wurden von MRS-Agarplatte beimpft, 17 h bei 37°C, 200 Upm inkubiert und zum Fermentationsmedium gegeben. Die Fermentation wurde bei 37°C, 0,1 bar Innendruck, pH 5,8 mit einer Begasungsrate von 1 l/min und 100 Upm gestartet (keine p $O_2$- Regelung) und nach 23 h bei einer OD600 von 14,8 beendet.
Die Aktivität der gewaschenen Zellproben wurde analog zu Beispiel 4a) mit ruhenden Zellen in MES, pH6.0 bestimmt.

Die Reinigung der Dehydrogenase aus Lu10288 wurde wie folgt durchgeführt:

Homogenisation:

100g Biofeuchtmasse von Lu10288 (Lactobacillus brevis) wurden in 5 x 20g Portionen mit 100ml MES-Puffer, 1mM MgCl2, pH 7,1 resuspendiert und in einer Kugelmühle mit Glasperlen (0,1 mm-0,2mm Durchmesser, 50ml resuspendierte Zellen zu 50ml Glasperlen) in 10 Portionen für 20 Minuten unter Eiskühlung bei 4000rpm homogenisiert.
Die Glaskugeln wurden über eine G2 Glasnutsche abgetrennt und mit 20ml Puffer gewaschen. Das gesammelte Homogenat wurde dann in einem GSA Rotor bei 12000rpm für 20 Minuten geklärt (610ml).

a) Q-Sepharose Ionenaustausch-Chromatographie:

Eine Q-Sepharose fast flow (Pharmacia) mit einem Durchmesser von 5cm und einem Volumen von 400 ml wurde in 20 mM MES-Puffer, 1 mM $MgCl_2$, pH 6,8 äquilibriert. 610 ml Homogenat wurden mit 11 Tabletten Complete (Proteaseinhibitor-Mischung, Roche, Complete, EDTA-free; Cat. No.:1873580)

versetzt und auf die Q-Sepharose-Säule bei einer Auftragsgeschwindigkeit von 10ml/min aufgetragen. Die Detektion bei 280 nm. Dann wurde im gleichen Puffer mit drei Säulenvolumina gewaschen. Zur Elution wurde ein linearer Gradient von 20 mM MES, 1 mM $MgCl_2$, 1 M NaCl, pH 6,8 angelegt (in 100 Minuten von 0% NaCl nach 100% NaCl). Es wurden 10 ml Fraktionen gesammelt und getestet. Fraktion 42 bis 62 war im HPLC-Test mit 3-Chlor-1-(thien-2-yl)-propan-1-on aktiv.

b) Superdex Molekularsieb-Chromatographie:

Eine Superdex 200 Molekularsiebsäule (Pharmacia) mit einem Durchmesser von 2,6 cm und einem Volumen von 240 ml wurde in 20 mM MES, 1 mM $MgCl_2$, 1Tablette Complete pro Liter Puffer (EDTA frei), pH 7,1 äquilibriert. Die Wertfraktionen der Q-Sepharose wurden vereinigt (240ml) und durch langsame Zugabe von 124 g Ammoniumsulfat auf 80 %ige Sättigung eingestellt. Der pH wurde auf 7,1 gehalten. Die Lösung wurde 10 Minuten bei 4°C gerührt und dann 20 Minuten bei 12000 U/min abzentrifugiert. Das erhaltene Pellet wurde in 5 ml Äquilibrierungspuffer resuspendiert und für 1 Stunde bei 4 Grad Celsius dialysiert (10 kDa Ausschlußvolumen). Die dialysierte Lösung wurde in zwei Teile ä 9 ml aufgeteilt und auf die Molekularsiebsäule bei einem Fluß von 4ml pro Minute aufgetragen. 4 ml Fraktionen wurden gesammelt und getestet. Fraktionen 48 bis 56 waren wieder für beide Substrate aktiv.

c) Mono-Q Ionenaustausch-Chromatographie:

Eine präparative Mono-Q HR Säule (Pharmacia) mit einem Volumen von 20 ml wurde in 20 mM MES, 1mM $M_9Cl_2$, pH 7,1 äquilibriert. 70 ml der vereinigten Wertfraktionen der Superdex wurden mit einer Flussgeschwindigkeit von 4 ml pro Minute aufgetragen. Nach dem Waschen der Säule wurde die Säule mit einem linearen Gradienten in 100 Minuten nach 100% 20 mM MES, 1 mM $MgCl_2$, 0,5 M NaCl , pH 7,1 entwickelt. Es wurden 4 ml Fraktionen gesammelt. Aktive Fraktionen (Fraktion 36 bis 41) wurden vereinigt.

d) Mono-P Ionenaustausch-Chromatographie:

Eine Mono-P Säule (Pharmacia, 4 ml) wurde in 20 mM MOPS, 1mM $MgCl_2$, pH 7,1 äquilibriert. 21 ml der Mono-Q Wertfraktion wurde bei einem Fluß von 0,75 ml/min aufgetragen. Nach dem Waschen bis zur Basislinie wurde ein linerarer Gradient in 10O Minuten nach 100% 20 mM MOPS, 1 mM $MgCl_2$, 0,5 M NaCl, pH 7,1 angelegt. Fraktionen (0,75ml) wurden gesammelt. Aktive Fraktionen (34-39) wurden vereinigt.

Aktivitätsfärbung in Gelen:

Die Probe wurde mit dem gleichen Volumen Novex "Native Tris-Glycin" Probenpuffer (Novex) verdünnt. Die Tris-Glycin-Gele von Anamed (ohne SDS, 1 mm dick, 10 Probentaschen) wurden in die Laufkammer eingebaut. Als Laufpuffer wurde Invitrogen "Tris-Glycin Native" Laufpuffer (Invitrogen) (10x) nach Verdünnung eingesetzt. Die Probentaschen wurden beladen und das Gel bei 200 V und ca 50 mA gestartet. Die Dauer der Elektrophorese betrug etwa 1,5 Stunden. Die Laufkammer stand in Eiswasser. Nachdem das Gel ausgebaut wurde kam es in eine Glasschale und wurde in 50mM MES, 8mM $MgCl_2$, pH 6,2 gewaschen und inkubiert.

Dann wurde zu diesem Gel in dieser Lösung 0,35mM NADP und 0,35mM NAD, 19,6mg NB-Tetrazolium und 2,1 mg Phenazin Ethosulfat (PES) gegeben. Dann erfolgte die Zugabe des Substrates (3-Chlor-1-(thien-2-yl)-propan-1-ol) ad 1 mM Endkonzentration. Die Gele standen bis zur Färbung in Dunkelheit. Ein typisches Gel ist in Figur 1 gezeigt.

**Bilanz der Isolierung von LU 10288 Dehydrogenase**

| Bezeichnung | Volumen [ml] | Protein [mg/ml] | Aktivität [U/l] | spezifische Aktivität | ee-Wert (S) | Protein gesamt | Aktivität gesamt | Ausbeute Protein | Ausbeute Aktivität |
|---|---|---|---|---|---|---|---|---|---|
| | | | | [mU/mg] | [%] | [mg] | [U] | [%] | in [%] |
| Zellsuspension | 500 | | 270,1 | | 95,0 | 0,0 | 135,1 | - | 100,0 |
| Homogenat | 610 | 3,35 | 98,1 | 29,3 | 94,1 | 2043,5 | 59,6 | 100,0 | 44,3 |
| WP-Q-Sepharose | 240 | 2,25 | 49,3 | 21,9 | 97,0 | 540,0 | 11,8 | 26,4 | 8,8 |
| WP-Superdex direkt best.* | 70 | 2,17 | 134 | 61,8 | 45,3 | 151,9 | 9,38 | 7,4 | 6,95 |
| WP-Superdex 8 Tage 4°C** | 70 | 2,17 | 6,4 | 2,9 | 62,4 | 151,9 | 0,45 | 7,4 | 0,33 |
| WP- Mono Q | 21 | 0,31 | 9,6 | 31,0 | 67,1 | 6,5 | 0,20 | 0,32 | 0,15 |
| WP-Mono P | 4,2 | 0,91 | 29,8 | 32,7 | 71,3 | 3,8 | 0,13 | 0,19 | 0,09 |

Wp: Wertpeak-Fraktionen
* Aktivitätbestimmung der frischen Probe
** Aktivitätbestimmung nach Lagerzeit

Der N-Terminus der so gereinigten Dehydrogenase wurde nach SDS-PAGE und Gelblotting mittels Edman-Sequenzierung bestimmt (SEQ ID NO: 1).

**Beispiel 6:** Reinigung der Dehydrogenase aus Candida magnoliae

Zur Fermentation von *Candida magnoliae* Lu8742 wurde 2 Vorkulturen in 150 ml Medium mit 8 g/fl Hefeextrakt (65%), 5 g/l Pepton, 3,5 g/l Glucose, 5 g/l $KH_2PO_4$ , 2 g/l $MgSO_4$ * $7H_2O$, pH6,0 15 Stunden bei 28 °C und 200 Upm angezogen und in 13,2 l analoges Medium mit 10,7 g/l Glucose und 4 ml Tegosipon überimpft. Die Fermentation wurde bei 28°C, 0,1 bar Innendruck, pH 6,0 mit einer Begasungsrate von 5 l/min und 500 Upm gestartet (Regelung $pO_2$ > 20%) und nach 26 h bei einer OD600 von 15,1 beendet.

Homogenisation:

Die geernteten Zellen (378 g Biofeuchtmasse) wurden in 1 l 50 mM MES +1 mM $MgCl_2$, pH 6,5 mit 10Tabletten Protease-Inhibitor Complete(ohne EDTA), Fa. Roche, suspendiert (ca. 9 x Konz.) und 2 x mit 1000 bar in einem Microfluidizer Z04 aufgeschlossen. Nach Zentrifugation (20 min/10000 g) wurde die Hälfte des klaren Überstandes (1,3 l Homogenat) wie nachfolgend beschrieben gereinigt. Dabei wurden die Fraktions-Proben in geeigneter Verdünnung in 50 mM MES pH 6,0, 0,2 mM NaDH/NaDPH, 100 mM Glucose, 50 µl/ml GDH und 10 mM 3-Chlor-1-(thien-2-yl)-propan-1-on bei 30°C auf Aktivität getestet.

Q-Sepharose Ionenaustausch-Chromatographie:

Eine Q-Sepharose fast flow (Pharmacia) mit einem Durchmesser von 5 cm und einem Volumen von 400 ml wurde in 20 mM MES-Puffer, 1 mM $MgCl_2$, pH 6,8 äquilibriert. 650 ml Homogenat wurden mit 11 Tabletten Complete (Proteaseinhibitor Mischung) versetzt und auf die Q-Sepharose-Säule bei einer Auftragsgeschwindigkeit von 7,5 ml/min aufgetragen. Die Detektion erfolgte bei 280 nm. Dann wurde mit 700 ml gleichem Puffer gewaschen. Zur Elution wurde ein linearer Gradient von 20 mM MES, 1 mM $MgCl_2$, 0,75 M NaCl, pH 6,8 angelegt (in 100 Minuten von 0% NaCl nach 100% NaCl) und mit 200 ml 20 mM MES, 1 mM $MgCl_2$, 0,75M NaCl, pH 6,8 nachgewaschen. Es wurden 10 ml Fraktionen gesammelt und getestet. Fraktion 56 bis 96 war im HPLC-Test mit 3-Chlor-1-(thien-2-yl)-propan-1-on aktiv.

Ammoniumsulfat-Fällung:

41 ml Q-Sepharose-Wertpeak-Fraktionen wurden ad 90 % mit $(NH_4)_2SO_4$ gesättigt (pH 6,2), 30 min bei 4°C gerührt und anschließend 10 min bei 10000 g zentrifugiert. Das erhaltene Pellet wurde in 10 ml 20 mM MES, 1 mM $MgCl_2$, 1 Tablette/l Complete(ohne EDTA), Fa. Roche, pH 6,2 suspendiert und 30 min gegen 20 mM MES, 1 mM $MgCl_2$, 1 Tablette/l Complete(ohne EDTA), Fa. Roche, pH 6,2, in einem Slide-A-Lyzer, Fa. Pierce (10 kDa Ausschlussvolumen), dialysiert.

Superdex Molekularsieb-Chromatographie:

Eine Superdex 200 Molekularsiebsäule (Pharmacia) mit einem Durchmesser von 2,6 cm und einem Volumen von 240 ml wurde in 20 mM MES, 1 mM $MgCl_2$, 1 Tablette Complete pro Liter Puffer (EDTA frei), pH 6,2 äquilibriert. Das Dialysat aus der Ammoniumsulfatfällung (2 x 7ml) wurde auf die Molekularsiebsäule bei einem Fluß von 4 ml pro Minute aufgetragen. 4 ml Fraktionen wurden gesammelt und getestet. Fraktionen 21 bis 24 waren wieder für beide Substrate aktiv.

Mono-Q Ionenaustausch-Chromatographie:

Eine Mono-Q HR5/5 Säule (Fa. Pharmacia) mit einem Volumen von 1 ml wurde in 20 mM MES, 1 mM $MgCl_2$, pH 6,8 äquilibriert. 10ml der vereinigten Wertfraktionen der Superdex-Säule wurden mit einer Flussgeschwindigkeit von 1ml pro Minute aufgetragen. Nach dem Waschen der Säule wurde die Säule mit einem linearen Gradienten in 100 Minuten nach 100% 20 mM MES, 1 mM $M_9Cl_2$, 0,75 M NaCl, pH 6,8 entwickelt und 10 min mit 20 mM MES, 1 mM $M_9Cl_2$, pH 6,8 nachgespült. Es wurden 1 ml Fraktionen gesammelt (Detektion 226 nm). Aktive Fraktionen (Fraktion 24 bis 27) wurden vereinigt.

Das Ergebnis der Isolierung ist in folgender Tabelle zusammengefasst

| Probe | Vol. [ml] | Aktivität [U/l] | Gesamt-aktivitäts[mU] | Aktivität-ausbeute [%] | Protein [g/L] | Gesamtprotein [mg] | Proteinausbeute [%] | ee(*) | spez.Akt. [U/g Protein] |
|---|---|---|---|---|---|---|---|---|---|
| Homogenat | 650 | 30 | 19500 | 100,0% | 5,70 | 3705 | 100 | 72,1 | 5,3 |
| Q-Wp | 54 | 42,8 | 2310 | 11,8% | 3,58 | 193 | 5,2 | 84,6 | 11,9 |
| AS-Fäll. | 18 | 217,2 | 4005 | 20,5% | 5,49 | 101 | 2,7 | 91,3 | 39,6 |
| Superde x-Wp | 49 | 35,6 | 1733 | 8,9% | 0,33 | 16,1 | 0,43 | 91,6 | 107,7 |
| MonoQ-Wp | 12 | 12,5 | 148 | 0,8% | 0,05 | 0,44 | 0,01 | 97,0 | 253,0 |

* (S)-3-Chlor-1-(thien-2-yl)-propan-1-ol
AS-Fäll.: Ammoniumsulfat-Fällung
Wp: Wertpeak-Fraktionen

Der N-Terminus der so gereinigten Dehydrogenase wurde nach SDS-PAGE und Gelblotting mittels Edman-Sequenzierung bestimmt (SEQ ID NO: 2).

**Beispiel 7:** Herstellung von (S)-3-Methylamino-1-(thien-2-yl)-propan-1-ol I-S durch Reduktion mit einer Dehydrogenase aus Lactobacillus brevis

Lactobacillus brevis Lu10288 wurde wie in Beispiel 4 angezogen und geerntet. Die ruhenden Zellen (10-100 g/L Biomasse) wurden mit 0,2 - 2 mM $NAD(P)^+$, 1 bis 100 g/L des Propanons aus Beispiel 1 (batch oder fed-batch) und 18 g/L Glucose versetzt und 2-8 h bei 30 °C inkubiert. Die Reaktion wurde durch Titration mit 0,5 M NaOH bei pH 6,0-7,0 gehalten und durch HPLC-Analytik verfolgt. Figuren 2A und B zeigen typische Verläufe gemäß den Ansätzen 1 bzw.2.

Ansatz 1:

| | |
|---|---|
| 9 ml | Zellsuspension Lu10288 (mit 200g BFM / l) |
| 3 ml | 1 M Glukose Lösung |
| 3 ml | 20 mM NADP Lösung |
| 0,9 ml | GDH Lösung (12-15 U/μl) |
| 3 ml | 1 M NaCl Lösung |
| 11,1 ml | Wasser |
| + 10 mM | Keton (aus Beispiel 1) |
| Nach 1 h wurden erneut 10 mM Keton zugegeben. | |

Ansatz 2:

| | |
|---|---|
| 9 ml | Zellsuspension Lu10288 (200g BFM / l) |
| 3 ml | 1 M Glukose-Lösung |
| 3 ml | 2 mM NADP-Lösung |
| 3 ml | 2 mM NAD-Lösung |
| 0,9 ml | GDH Lösung (12-15 U/μl) |
| 3 ml | 1 M NaCl Lösung |
| 11,1 ml | Wasser |
| + 10 mM | Keton (0,6 ml 0,5 M Keton aus Beispiel 1 in Methanol) |
| Nach 45, 110, 180 und 300 min wurden erneut je 10 mM Keton zugegeben. | |

Im Anschluß wurde die Biomasse durch Zentrifugation und/oder Filtration entfernt. Die NMR-Analyse der MTBE-Extrakte ergab einen 3-Chlor-1-(thien-2-yl)-propan-1-ol-Gehalt von 60-70%. Die Gehalte an nicht umgesetztem 3-Chlor-1-(thien-2-yl)-propan-1-on sowie an dem Nebenprodukt 1-Thien-2-yl-propen-1-on lagen je unter 10%. 1-Thien-2-yl-propen-1-ol konnte nur in Spuren detektiert werden.

Zu dem zellfreien wässrigen Ansatz gab man anschließend 1,1 g 40%ige wässrige Methylaminlösung zu. Das Gemisch wurde 6 h bei 60 °C unter Eigendruck gerührt. Nach dem Abkühlen auf Raumtemperatur entfernte man das Lösungsmittel, digerierte den Rückstand mit Toluol und filtrierte davon ab. Alternativ konnte die Zellabtrennung nach der Aminierungsreaktion durchgeführt werden. Nach dem Trocknen des Filtrats erhielt man 202 mg der Titelverbindung in Form eines hellgelben Feststoffs. Man erhielt das S-Enantiomer in einem Enantiomerenüberschuss von 95 % ee. Die Bestimmung des ee-Wertes erfolgte durch Bestimmung des Drehwertes (c = 1, Lösungsmittel: Methanol) und mittels Shift-NMR (Shift-Reagenz: 2,2,2-Trifluor-1-(9-anthryl)ethanol (+) (TFAE) ; Lösungsmittel: $CDCl_3$; 500 MHz)

**Beispiel 8:** Klonierung der Dehydrogenase aus Lactobacillus brevis Lu10288

a) Chromosomale DNA-Präparation aus LU10288 nach vorheriger Protoplastierung:

<u>(1) Benötigte Lösungen</u>

| | |
|---|---|
| **Lösung1:** | 0,41 M Saccharose |
| | 0,01 M $MgSO_4*7H_2O$ |
| | 5ml/l M12-Medium 1:2 |
| | 10ml/l 10% $KH_2PO_4$ pH 6,7 |
| | 2,5 mg/ml Lysozym (kurz vor Gebrauch zugeben) |

Ansetzen der Lösung 1 wie folgt:
14,03 g Saccharos, 0,25 g $MgSO_4*7H_2O$, 5ml M12 (10x conc) und 1 ml 10% $KH_2PO_4$ pH 6,7 auf 100 ml auffüllen und sterilfiltrieren.

| | |
|---|---|
| **Proteinase:** | Fa. Qiagen, 20mg/ml stock solution |
| **RNase:** | Fa. Qiagen, 100mg/ml stock solution |
| **TE-Puffer:** | 10mM Tris*Cl pH8, 1 mM EDTA pH 8 |

(2) Anzucht und Aufschluß:

- Anzucht über Nacht in 100ml MRS- Medium (Fa. Difco) in 250ml Erlenmeyerkolben mit Schikanen bei 37°C und 200 rpm.
- Zellen zentrifugieren: 4000 rpm, 10 min, 4°C
- Überstand verwerfen und das Pellet in 5ml Lösung1(+ Lysozym) aufnehmen und gut resuspendieren. Im Brutschrank (37°C) 1 - 4h inkubieren.
- Protoplasten vorsichtig zentrifugieren: 3000 rpm, 10 min.
- Überstand verwerfen, Pellet mit 10 ml Lösung1 (ohne Lysozym) waschen: 3000 rpm, 4°C, 8 min.
- Überstand verwerfen, Pellet mit 10 ml 0,01 M Tris-HCl pH 8,0 waschen: 3000rpm, 4°C, 8 min.
- Überstand verwerfen, Pellet in 4 ml TE - Puffer resuspendieren.
- Zugabe von 0,5 ml 10% SDS und 0,5 ml 5M NaCl, vorsichtig mischen.
- Zugabe von 1 mg Proteinase K (Qiagen Proteinase: 20 mg/ml, also 50 μl) und Inkubation bei 37°C, über Nacht im Brutschrank.
- Diesen Ansatz mit TE - Puffer auf 20 ml auffüllen.

(3) Extraktion:

- Zugabe von Phenol 1:1, d.h. 20ml Phenol + 20 ml Ansatz. Vorsichtig mischen und bei 4°C, 4000 rpm, 5min zentrifugieren.
- Obere Phase abheben und in neues Falcon (20ml) überführen.
- Zugabe von 20ml Phenol:Chloroform:Isoamylalkohol (25:24:1). Vorsichtig mischen und bei 4°C, 4000rpm, 5min zentrifugieren.
- Obere Phase abheben und in neues Falcon (ca. 18ml) überführen.
- Zugabe von 18 ml Chloroform:Isoamylalkohol (24:1, also 18ml Chloroform+333μl Isoamylalkohol). Vorsichtig mischen und bei 4°C, 4000 rpm, 5min zentrifugieren. Diesen Schritt so lang wiederholen, bis obere Phase klar ist.
- Obere Phase (18ml) mit 2 Volumen Ethanol (36ml) fällen. Zugabe von 1/50 3M Natriumacetat (ca. 360μl). Mindestens 30min bei -20°C fällen lassen. Danach bei 4°C, 12000 rpm, 30min zentrifugieren.
- Überstand verwerfen, Pellet in 1-2ml TE-Puffer aufnehmen. Zugabe von 20μg RNase pro ml TE-Puffer. Inkubation: 1 h, 37°C im $H_2O$-Bad.

(4) Dialyse:

Nach RNase-Behandlung füllt man den Ansatz in einen Dialyseschlauch. Es wird 3mal jeweils für 1 Stunde bei 4°C in 1,51 TE-Puffer dialysiert. Der letzte Dialyseschritt ist auch über Nacht möglich.

- Dialysierte DNA in Falcon füllen und auf mehrere Eppendorff-Gefäße verteilen (500μl).

- Zugabe von 2 Volumen Ethanol (1000μl) und 1/3 Volumen 2M LiCl (166μl).
- Fällung bei -20°C, mindestens 30min. Danach bei 4°C, 12000 rpm, 30min zentrifugieren.
- Überstand vorsichtig abschütten.
- Waschen des Pellets mit 20ml 70% Ethanol und bei 12000 rpm, 4°C, 15min zentrifugieren.
- Überstand vorsichtig abschütten, das Pellet an der Luft trocknen lassen und die DNA in entsprechendem Volumen 10mM Tris*HCl pH 8,0 aufnehmen (je nach Pelletgröße ab 100 μl).

Zur Verbesserung des Rücklösens wurde die DNA 1-2 Stunden im Eppendorfschüttler bei 55-60°C bei leichter Schüttelfrequenz (400 rpm) inkubiert.

b) Aus der Proteinreinigung des Beispiels 5 wurden nach tryptischem Verdau und Edmannsequenzierung der Peptide weitere Aminosäuresequenzen erhalten. Ergebnisse der Sequenzanalyse sind in Fig. 3B zusammengestellt.

Die Sequenz FVVDGGYTAQ (vgl. V8-RP Fr.7) stellt aufgrund des Abbruchs vermutlich den C-Terminus dar. Von dieser und von der N-terminalen Aminosäuresequenz (SEQ ID NO. 1) wurden unter Berücksichtigung der Codon Usage von Lactobacillus brevis Nukleinsäuresequenzen abgeleitet (Primer Mke338 und 339), die wie folgt der Klonierung des Dehydrogenase-Gens durch PCR-Amplifikation an chromosomaler DNA aus Lu10288 (Protokoll siehe oben) dienten.

PCR:

| Template | Primer | Produkt Länge |
|---|---|---|
| Chromosomale DNA aus Lu10288 * | Mke338+Mke339 | ca. 800 bp |
| * hergestellt gemäß Beispiel 8a) | | |

Primer:

| **Primer Nr.** | **Sequenz (5'-3')** | **Position** |
|---|---|---|
| Mke338 | GGGAATTCCATATGTCTAACCGTTTGG | N-Term-Primer (NdeI) |
| Mke339 | CGTAGGGAAGCTTATTGAGCAGTGTAGC | C-Term-Primer (HindIII) |

Die PCR wurde nach Stratagene-Standardvorschrift mit Pfu-Polymerase (Stratagene) und dem folgenden Temperatur-Programm durchgeführt: 95°C für 5 Minuten; 30 Zyklen mit 95°C für 45 sec., 52°C für 45 sec, und 72°C für 1 min 20 sec; 72°C für 10 min.; 10°C bis zur Verwendung. Das PCR-Produkt (0,8 kB) wurde durch Agarosegel-Electrophorese (1,2%E-Gel, Invitrogen) und Säulen-Chromatographie (Mini-Elute, Qiagen) isoliert und anschließend mit NdeI/HindIII verdaut und in entsprechend verdauten pDHE19.2-Vektor (ein pJOE-Derivat, DE19848129) kloniert. Die Ligationsansätze wurden in E.coli XL1 Blue (Stratagene) transformiert. Die Sequenzierung entsprechender Klone ergab als Insert im so erhaltenen Plasmid pDHE10288adh1 die in SEQ ID NO:3 dargestellte Nukleinsäuresequenz, die der Aminosäuresequenz SEQ ID NO:4 entspricht. In ihr finden sich alle in Beispiel 5 und 8 identifizierten Peptide wieder.
SEQ ID NO: 4 Aminosäuresequenz der Dehydrogenase aus Lu10288

```
  1  MSNRLDGKVA IVTGGTLGIG LAIATKFVEE GAKVMITGRH SDVGEKAAKS
 51  VGTPDQIQFF QHDSSDEDGW TKLFDATEKA FGPVSTLVNN AGIAVNKSVE
101  ETTTAEWRKL LAVNLDGVFF GTRLGIQRMK NKGLGASIIN MSSIEGFVGD
151  PSLGAYNASK GAVRIMSKSA ALDCALKDYD VRVNTVHPGY IKTPLVDDLP
201  GAEEAMSQRT KTPMGHIGEP NDIAYICVYL ASNESKFATG SEFVVDGGYT
251  AQ*
```

SEQ ID NO: 3 Nukleinsäuresequenz der Dehydrogenase aus Lu10288 (sowie Gegenstrang)

```
  1 ATGTCTAACC GTTTGGATGG AAAAGTAGCA ATCGTTACAG GTGGTACGTT
    TACAGATTGG CAAACCTACC TTTTCATCGT TAGCAATGTC CACCATGCAA
 51 GGGTATCGGT TTAGCTATCG CCACGAAGTT CGTTGAAGAA GGGGCTAAGG
    CCCATAGCCA AATCGATAGC GGTGCTTCAA GCAACTTCTT CCCCGATTCC
101 TCATGATTAC CGGCCGGCAC AGCGATGTTG GTGAAAAAGC AGCTAAGAGT
    AGTACTAATG GCCGGCCGTG TCGCTACAAC CACTTTTTCG TCGATTCTCA
151 GTCGGCACTC CTGATCAGAT TCAATTTTTC CAACATGATT CTTCCGATGA
    CAGCCGTGAG GACTAGTCTA AGTTAAAAAG GTTGTACTAA GAAGGCTACT
201 AGACGGCTGG ACGAAATTAT TCGATGCAAC GGAAAAAGCC TTTGGCCCAG
    TCTGCCGACC TGCTTTAATA AGCTACGTTG CCTTTTTCGG AAACCGGGTC
251 TTTCTACATT AGTTAATAAC GCTGGGATCG CGGTTAACAA GAGTGTCGAA
    AAAGATGTAA TCAATTATTG CGACCCTAGC GCCAATTGTT CTCACAGCTT
301 GAAACCACGA CTGCTGAATG GCGTAAACTA TTAGCCGTCA ACCTTGATGG
    CTTTGGTGCT GACGACTTAC CGCATTTGAT AATCGGCAGT TGGAACTACC
351 TGTCTTCTTC GGTACCCGAT TAGGGATTCA ACGGATGAAG AACAAAGGCT
    ACAGAAGAAG CCATGGGCTA ATCCCTAAGT TGCCTACTTC TTGTTTCCGA
401 TAGGGGCTTC CATCATCAAC ATGTCTTCGA TCGAAGGCTT TGTGGGTGAT
    ATCCCCGAAG GTAGTAGTTG TACAGAAGCT AGCTTCCGAA ACACCCACTA
451 CCTAGCTTAG GGGCTTACAA CGCATCTAAA GGGGCCGTAC GGATTATGTC
```

```
    GGATCGAATC CCCGAATGTT GCGTAGATTT CCCCGGCATG CCTAATACAG
501 CAAGTCAGCT GCCTTAGATT GTGCCCTAAA GGACTACGAT GTTCGGGTAA
    GTTCAGTCGA CGGAATCTAA CACGGGATTT CCTGATGCTA CAAGCCCATT
551 ACACTGTTCA CCCTGGCTAC ATCAAGACAC CATTGGTTGA TGACCTACCA
    TGTGACAAGT GGGACCGATG TAGTTCTGTG GTAACCAACT ACTGGATGGT
601 GGGGCCGAAG AAGCGATGTC ACAACGGACC AAGACGCCAA TGGGCCATAT
    CCCCGGCTTC TTCGCTACAG TGTTGCCTGG TTCTGCGGTT ACCCGGTATA
651 CGGTGAACCT AACGATATTG CCTACATCTG TGTTTACTTG GCTTCTAACG
    GCCACTTGGA TTGCTATAAC GGATGTAGAC ACAAATGAAC CGAAGATTGC
701 AATCTAAATT TGCAACGGGT TCTGAATTTG TAGTTGACGG TGGCTACACT
    TTAGATTTAA ACGTTGCCCA AGACTTAAAC ATCAACTGCC ACCGATGTGA
751 GCTCAA
    CGAGTT
```

**Beispiel 9:** Aktivitätsbestimmung der rekombinanten Dehydrogenase aus Lactobacillus brevis Lu10288

Das Plasmid pDHE10288adh1 wurde in *E.coli* TG10 pAgro4 pHSG575 retransformiert (TG10: ein RhaA$^-$-Derivat von E.coli TG1(Stratagene); pAgro4: Takeshita, S; Sato, M; Toba, M; Masahashi, W; Hashimoto-Gotoh, T (1987) Gene 61, 63-74; pHSG575: T. Tomoyasu et al (2001), Mol. Microbiol. 40(2), 397-413).

Je 6 Transformanten wurden in 20mL LBAmp/Spec/Cm (100µg/l Amp; 50mg/lSpec; 10µg/lCm) 0,1 mM IPTG 0,5g/L Rhamnose in 100mL Erlenmeyerkolben (Schikanen) 18 h bei 37°C angezogen, bei 5000g/10min zentrifugiert, einmal mit 10mM Tris/HCl pH7,0 gewaschen und in 2 ml des gleichen Puffers resuspendiert. 100 µl Zellsuspension wurden 20 min in 900 µl 50mM MES pH6 mit 50µl/ml Glucose-DH (Beispiel1), 10mM Glucose, 10mM NaCl, 1 mM NADH, 1 mM NADPH und mit 10 mM 3-Chlor-1-(thien-2-yl)-propan-1-on schüttelnd inkubiert. Die Ansätze wurden analog

zu Beispiel 4 analysiert. Im Durchschnitt wurden 0,13 mM 3-Chlor-1-(thien-2-yl)-propan-1-ol gebildet, was einer Aktivität von 6,6 U/L Kultursuspension entspricht. In analogen Ansätzen mit Rohextrakt, der durch Zellaufschluß mit 0,7ml Glaskugeln (d=0,5mm) in einer Schwingmühle (3x 5min mit Zwischenkühlung auf Eis) gewonnen wurde, wurden 0,21 mM 3-Chlor-1-(thien-2-yl)-propan-1-ol, entsprechend einer Aktivität von 10,7 U/L, gemessen. In Kontrollversuchen ohne Zusatz von Rhamnose bei der Anzucht war kein 3-Chlor-1-(thien-2-yl)-propan-1-ol detektierbar.

**Beispiel 10:** Klonierung der Dehydrogenase aus Candida magnoliae Lu8472

Aus der Proteinreinigung des Beispiels 6 wurden nach nochmaliger Bestimmung der N-terminalen Sequenz durch Edmannsequenzierung folgende Aminosäuresequenz erhalten:
(S,G oder T)(T oder P)TSNALVTGGSRGIGAA

Nach tryptischem Verdau und Edmannsequenzierung der Peptide wurde folgende weitere Aminosäuresequenz erhalten:
IGVNSINPG

Von den Peptiden wurden unter Berücksichtigung der Codon Usage von Candida magnoliae Nukleinsäuresequenzen abgeleitet (Primer Mke366, 367 und 374), die wie folgt der Klonierung des Dehydrogenase-Gens durch PCR-Amplifikation an chromosomaler DNA aus Lu8472 (Genomic DNA Kit mit dreifach konzentrierter Lyticase-Lösung, Qiagen, Hilden) dienten.

PCR:

| Template | Primer | Produkt Länge |
|---|---|---|
| Chromosomale DNA aus Lu8472 | Mke366/367+ Mke374 | ca. 480 bp |

Primer:

| **Primer Nr.** | **Sequenz (5'-3')** | **Position** |
|---|---|---|
| Mke366 | ACGACGACGAGCAACGCBCTBGTBACGG | N-Term-Primer |
| Mke367 | ACGACGACGTCGAACGCBCTBGTBACGG | N-Term-Primer |
| Mke374 | GCCGGGGTTGATSSWGTTSACGCCGAT | C-Term-Primer |

Die Primer MKe366 und MKe367 wurden 1:1 gemischt. Die PCR wurde nach Stratagene-Standardvorschrift mit PfuTurbo-Polymerase (Stratagene) und dem folgenden Temperatur-Gradienten-Programm durchgeführt: 95°C für 1 Minuten; 30 Zyklen mit 95°C für 1 min., X°C[1] für 45 sec, und 72°C für 2 min; 72°C für 10 min.; 10°C bis zur Verwendung. Das PCR-Produkt (~0,5 kB) wurde durch Agarosegel-Electrophorese (1,2%E-Gel, Invitrogen) und Säulen-Chromatographie (GFX-Kit, Amersham Pharmacia) isoliert und anschließend sequenziert (Sequenzierprimer: Mke 366 und Mke374). Die erhaltene Sequenz ist in SEQ ID NO:5 dargestellt. Die daraus abgeleitete Amiosäuresequenz (SEQ ID NO:6) zeigt eine Identität von 53% zur Carbonyl-Reduktase aus Candida magnoliae (WO200140450). Die nach Proteinreinigung ermittelten Peptidsequenzen sind mit geringfügigen Abweichungen enthalten. Unterschiede könnten

[1] Es wurden 12 Ansätze mit unterschiedlichen Annealingtemperaturen gefahren, von 25°C bis 45°C (Delta je ca. 2 °C). In allen Ansätzen entstand als Hauptprodukt in ähnlicher Konzentration eine 0,5 kB-Bande.

einerseits auf Sequenzierfehlern beruhen oder durch die Existenz mehrerer Isoenzyme in Candida magnoliae Lu8742 bedingt sein.

SEQ ID NO:6 Partielle Aminosäuresequenz der Dehydrogenase aus Lu8472

```
  1  NALVTGGSRG IGEATAIKLA EEGYSVTIAS RGLKQLEAVK AKLPIVKQGQ
 51  VHHVWQLDLS DVDAAAAFKG SPLPASRYDV LVSNAGVAQF SPFIEHAKQD
101  WSQMLAINLA APIALAQTFA KAIGDKPRNT PAHIVFVSSN VSLRGFPNIG
151  VNSITPG
```

SEQ ID NO:5 Partielle Nukleinsäuresequenz der Dehydrogenase aus Lu8472 (sowie Gegenstrang)

```
  1 AACGCGCTGG TGACGGGCGG CAGCCGCGGC ATTGGCGAAG CCACTGCCAT
    TTGCGCGACC ACTGCCCGCC GTCGGCGCCG TAACCGCTTC GGTGACGGTA
 51 TAAGCTCGCC GAGGAGGGCT ACAGCGTCAC GATTGCGTCT CGCGGCCTTA
    ATTCGAGCGG CTCCTCCCGA TGTCGCAGTG CTAACGCAGA GCGCCGGAAT
101 AGCAGCTCGA GGCTGTGAAG GCCAAACTAC CCATTGTGAA GCAGGGACAG
    TCGTCGAGCT CCGACACTTC CGGTTTGATG GGTAACACTT CGTCCCTGTC
151 GTTCACCACG TGTGGCAGCT TGATCTCAGT GATGTCGACG CTGCGGCCGC
    CAAGTGGTGC ACACCGTCGA ACTAGAGTCA CTACAGCTGC GACGCCGGCG
201 CTTCAAAGGG TCGCCGCTAC CTGCCAGCCG CTACGACGTG CTCGTCAGCA
    GAAGTTTCCC AGCGGCGATG GACGGTCGGC GATGCTGCAC GAGCAGTCGT
251 ATGCTGGCGT GGCCCAGTTT AGCCCGTTCA TCGAGCATGC GAAGCAGGAC
    TACGACCGCA CCGGGTCAAA TCGGGCAAGT AGCTCGTACG CTTCGTCCTG
301 TGGTCGCAGA TGCTTGCCAT CAATCTGGCG GCACCCATTG CGCTGGCCCA
    ACCAGCGTCT ACGAACGGTA GTTAGACCGC CGTGGGTAAC GCGACCGGGT
351 GACATTTGCT AAGGCCATTG GCGACAAGCC GCGCAACACA CCGGCCCACA
    CTGTAAACGA TTCCGGTAAC CGCTGTTCGG CGCGTTGTGT GGCCGGGTGT
401 TTGTGTTTGT CTCGTCGAAC GTCTCGTTGC GAGGCTTCCC GAACATCGGC
    AACACAAACA GAGCAGCTTG CAGAGCAACG CTCCGAAGGG CTTGTAGCCG
451 GTCAACTCCA TCACCCCCGG CA
    CAGTTGAGGT AGTGGGGGCC GT
```

## Patentansprüche

1. Verfahren zur Herstellung von (S)-3-Methylamino-1-(thien-2-yl)-propan-1-ol der Formel I-S

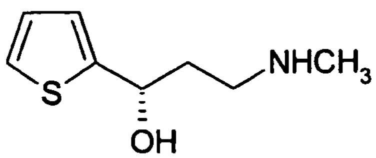

(I-S)

bei dem man

a) Thiophen mit einem β-Halogenpropionsäurehalogenid oder einem Acrylsäurehalogenid in Gegenwart einer Lewis-Säure zu einem 3-Halogen-1-(thien-2-yl)-propan-1-on umsetzt, wobei man gleichzeitig oder nach erfolgter Umsetzung, jedoch vor der Isolierung des Reaktionsprodukts, einen Halogenwasserstoff einleitet und
b) das in Schritt a) erhaltene Propanon in Gegenwart einer Dehydrogenase (E.C. 1.1.x.x.) reduziert, welche eine Selektivität bezüglich der Bildung von (S)-3-Methylamino-1-(thien-2-yl)propan-1-ol aufweist, und anschließend, gegebenenfalls ohne Isolierung des Reaktionsprodukts, mit Methylamin umsetzt.

**2.** Verfahren nach Anspruch 1, wobei man in Schritt a) als Lewis-Säure Aluminiumtrichlorid verwendet.

**3.** Verfahren nach einem der vorhergehenden Ansprüche, wobei man die Umsetzung in Schritt a) in einem halogenierten Kohlenwasserstoff als Lösungsmittel durchführt.

**4.** Verfahren nach Anspruch 1, wobei die Reduktion in Gegenwart einer Dehydrogenase (E.C. 1.1.1.x) insbesondere in Gegenwart einer Alkohol Dehydrogenase (E.C.1.1.1.1 oder E.C.1.1.1.2) durchgeführt wird.

**5.** Verfahren nach Anspruch 1 oder 4, wobei die Dehydrogenase ausgewählt ist unter Dehydrogenasen aus Hefen der Gattung Geotrichum, Pichia, Candida, Hansenula oder Saccharomyces und aus Bakterien der Gattung Pseudomonas, Burkholderia, Agrobacterium, Rhodococcus oder Lactobacillus.

**6.** Verfahren nach Anspruch 5, wobei die Dehydrogenase ausgewählt ist unter Dehydrogenasen aus Geotrichum candidum, Candida magnoliae und Lactobacillus brevis.

**7.** Verfahren nach Anspruch 1, wobei man eine Alkohol Dehydrogenase mit einer Aminosäuresequenz einsetzt, die im Bereich des N-Terminus

a) eine Aminosäureteilsequenz von wenigstens 10 aufeinanderfolgenden *Aminosäureresten* gemäß SEQ ID NO: 1 umfasst, wobei vorzugsweise zusätzlich die der Aminosäureposition 12 gemäß SEQ ID NO:1 entsprechende Position für Valin steht; oder eine
b) Aminosäureteilsequenz von wenigstens 10 aufeinanderfolgenden *Aminosäureresten* gemäß SEQ ID NO: 2 umfasst.

**8.** Verfahren nach Anspruch 7,wobei die Dehydrogenase zur Reduktion von 3-Chlor-1-(thien-2-yl)-propan-1-on zu (S)-3-Chlor-1-(thien-2-yl)-propan-1-ol befähigt ist.

**9.** Verfahren nach Anspruch 8, wobei die Dehydrogenase die Reduktion in einer Enantiomerenreinheit von wenigstens 85 % ee (in Gegenwart von NADH und/oder NADPH; bei 30°C und pH 6.0) katalysiert.

**10.** Verfahren nach einem der Ansprüche 7 bis 9, wobei die Dehydrogenase von einer Nukleinsäuresequenz, umfassend SEQ ID NO:3, kodiert wird, oder eine Aminosäuresequenz gemäß SEQ ID NO: 4 oder wenigstens eine Teilsequenz gemäß Figur 3 umfasst, und vorzugsweise aus Lactobacillus brevis erhältlich is oder eine davon abgeleitete funktional äquivalente Alkohol Dehydrogenase mit einer zu mindestens 60% identischen Aminosäuresequenz ist.

**11.** Verfahren nach einem der Ansprüche 7 bis 9, wobei die Dehydrogenase von einer Nukleinsäuresequenz, umfassend SEQ ID NO:5, kodiert wird, oder eine Aminosäuresequenz, umfassend SEQ ID NO: 6, aufweist, und vorzugsweise aus Candida magnoliae (ATCC 12573) erhältlich ist oder eine davon abgeleitete funktional äquivalente Alkohol Dehydrogenase mit einer zu mindestens 60% identischen Aminosäuresequenz ist.

**12.** Verfahren nach einem der Ansprüche 1 bis 11, wobei man die Dehydrogenase oder einen diese Dehydrogenase produzierenden natürlichen oder rekombinanten Mikroorganismus einsetzt.

## Claims

**1.** A process for preparing (S)-3-methylamino-1-(thien-2-yl)propan-1-ol of the formula I-S

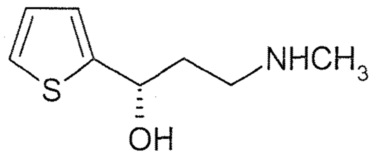

(I-S)

wherein

a) thiophene is reacted with a β-halopropionyl halide or an acryloyl halide in the presence of a Lewis acid to give a 3-halo-1-(thien-2-yl)propan-1-one, with a hydrogen halide being passed in simultaneously or after the reaction has taken place, but before the reaction product is isolated, and
b) the propanone obtained in step a) is reduced in the presence of a dehydrogenase (E.C. 1.1.x.x.) which exhibits selectivity with regard to the formation of (S)-3-methylamino-1-(thien-2-yl)propan-1-ol, and then, where appropriate without isolating the reaction product, reacted with methylamine.

**2.** The process according to claim 1, wherein the Lewis acid used in step a) is aluminum trichloride.

**3.** The process according to one of the preceding claims, wherein the reaction in step a) is carried out in a halogenated hydrocarbon as the solvent.

**4.** The process according to claim 1, wherein the reduction is carried out in the presence of a dehydrogenase (E.C. 1.1.1.x), in particular in the presence of an alcohol dehydrogenase (E.C.1.1.1.1 or E.C.1.1.1.2).

**5.** The process according to claim 1 or 4, wherein the dehydrogenase is selected from among dehydrogenases from yeasts of the genus Geotrichum, Pichia, Candida, Hansenula or Saccharomyces and from bacteria of the genus Pseudomonas, Burkholderia, Agrobacterium, Rhodococcus or Lactobacillus.

**6.** The process according to claim 5, wherein the dehydrogenase is selected from among dehydrogenases from Geotrichum candidum, Candida magnoliae and Lactobacillus brevis.

**7.** The process according to claim 1, wherein use is made of an alcohol dehydrogenase having an amino acid sequence which, in the region of the N terminus

a) comprises a constituent amino acid sequence of at least 10 consecutive *amino acid residues* as depicted in SEQ ID NO: 1, with the position corrresponding to amino acid position 12 as depicted in SEQ ID NO:1 preferably additionally standing for valine; or a
b) constituent amino acid sequence of at least 10 consecutive *amino acid resides* as depicted in SEQ ID NO: 2.

**8.** The process according to claim 7, wherein the dehydrogenase is capable of reducing 3-chloro-1-(thien-2-yl)propan-1-one to (S)-3-chloro-1-(thien-2-yl)propan-1-ol.

**9.** The process according to claim 8, wherein the dehydrogenase catalyzes the reduction in an enantiomeric purity of at least 85% ee (in the presence of NADH and/or NADPH; at 30°C and pH 6.0).

**10.** The process according to one of claims 7 to 9, wherein the dehydrogenase is encoded by a nucleic acid sequence comprising SEQ ID NO:3 or comprises an amino acid sequence as depicted in SEQ ID NO: 4 or at least a constituent sequence as depicted in Figure 3, and can preferably be obtained from Lactobacillus brevis or is a functionally equivalent alcohol dehydrogenase which is derived therefrom and has an amino acid sequence which is at least 60% identical.

**11.** The process according to one of claims 7 to 9, wherein the dehydrogenase is encoded by a nucleic acid sequence comprising SEQ ID NO:5 or possesses an amino acid sequence comprising SEQ ID NO: 6 and can preferably be obtained from Candida magnoliae (ATCC 12573) or is a functionally equivalent alcohol dehydrogenase which is derived therefrom and has an amino acid sequence which is at least 60% identical.

**12.** The process according to one of claims 1 to 11, wherein the dehydrogenase or a natural or recombinant microorganism which produces this dehydrogenase is used.

## Revendications

1. Procédé de préparation de (S)-3-méthylamino-1-(thién-2-yl)-propan-1-ol de formule I-S

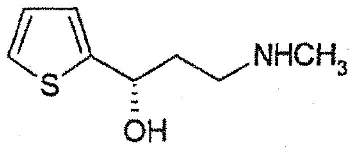

(I-S)

dans lequel

a) on fait réagir du thiophène avec un halogénure de β-halogénopropionyle ou un halogénure d'acryloyle en présence d'un acide de Lewis, pour donner une 3-halogéno-1-(thién-2-yl)-propan-1-one, avec introduction d'un acide halogènhydrique, en même temps que la réaction ou après cette dernière, mais avant isolement du produit de la réaction, et
b) on réduit la propanone obtenue dans l'étape a) en présence d'une déshydrogénase (E.C. 1.1.x.x.), qui présente une sélectivité vis-à-vis de la formation du (S)-3-méthylamino-1-(thién-2-yl)-propan-1-ol, puis, éventuellement sans isolement du produit de la réaction, on fait réagir avec de la méthylamine.

2. Procédé selon la revendication 1, dans lequel, dans l'étape a), on utilise en tant qu'acide de Lewis du trichlorure d'aluminium.

3. Procédé selon l'une des revendications précédentes, dans lequel on met en oeuvre la réaction de l'étape a) dans un hydrocarbure halogéné en tant que solvant.

4. Procédé selon la revendication 1, dans lequel on met en oeuvre la réduction en présence d'une déshydrogénase (E.C. 1.1.1.x), en particulier en présence d'une alcool déshydrogénase (E.C. 1.1.1.1. ou E.C. 1.1.1.2.).

5. Procédé selon la revendication 1 ou 4, dans lequel la déshydrogénase est choisie parmi les déshydrogénases de levures du genre Geotrichum, Pichia, Candida, Hansenula ou Saccharomyces, ou de bactéries du genre Pseudomonas, Burkholderia, Agrobacterium, Rhodococcus ou Lactobacillus.

6. Procédé selon la revendication 5, dans lequel la déshydrogénase est choisie parmi les déshydrogénases de Geotrichum candidum, Candida magnoliae et Lactobacillus brevis.

7. Procédé selon la revendication 1, dans lequel on utilise une alcool déshydrogénase ayant une séquence d'acides aminés qui, dans la zone du site N-terminal,

a) comprend une séquence partielle d'acides aminés constituée d'au moins 10 résidus d'acides aminés successifs selon SEQ ID N°1, dans laquelle au moins en outre la position correspondant à la position d'acide aminé 12 selon SEQ ID N°1 représente la valine ; ou
b) comprend une séquence partielle d'acides aminés d'au moins 10 résidus d'acides aminés successifs selon SEQ ID N°2.

8. Procédé selon la revendication 7, dans lequel la déshydrogénase est apte à réduire la 3-chloro-1-(thién-2-yl)-propan-1-one en (S)-3-chloro-1-(thién-2-yl)-propan-1-ol.

9. Procédé selon la revendication 8, dans lequel la déshydrogénase catalyse la réduction selon une pureté énantiomérique d'au moins 85 % ee (en présence de NADH et/ou de NADPH ; à 30°C et à pH 6,0).

10. Procédé selon l'une des revendications 7 à 9, dans lequel la déshydrogénase est codée par une séquence d'acide nucléique comprenant SEQ ID N°3, ou comprend une séquence d'acides aminés selon SEQ ID N°4 ou au moins une séquence partielle selon la Figure 3, et peut être obtenue de préférence à partir de Lactobacillus brevis, ou est une alcool déshydrogénase fonctionnellement équivalente, qui en dérive, avec une séquence d'acides aminés présentant une identité d'au moins 60 %.

**11.** Procédé selon l'une des revendications 7 à 9, dans lequel la déshydrogénase est codée par une séquence d'acide nucléique comprenant SEQ ID N°5, ou présente une séquence d'acides aminés comprenant SEQ ID N°6, et peut être obtenue de préférence à partir de Candida magnoliae (ATCC 12573), ou est une alcool déshydrogénase fonctionnellement équivalente, qui en dérive, ayant une séquence d'acides aminés présentant une identité d'au moins 60 %.

**12.** Procédé selon l'une des revendications 1 à 11, dans lequel on utilise la déshydrogénase ou un microorganisme naturel ou recombinant produisant cette déshydrogénase.

Fig. 1

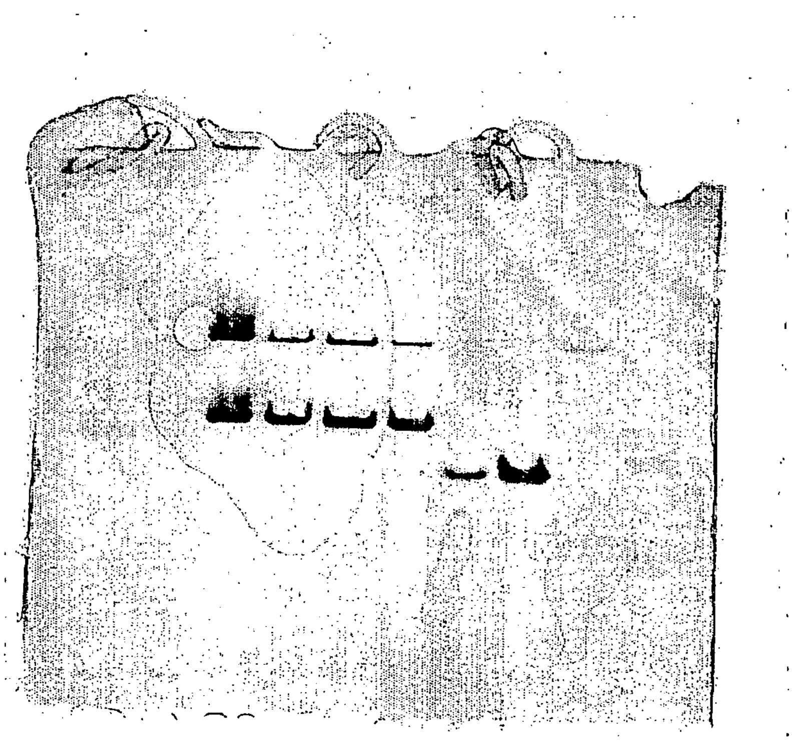

Fig. 2A

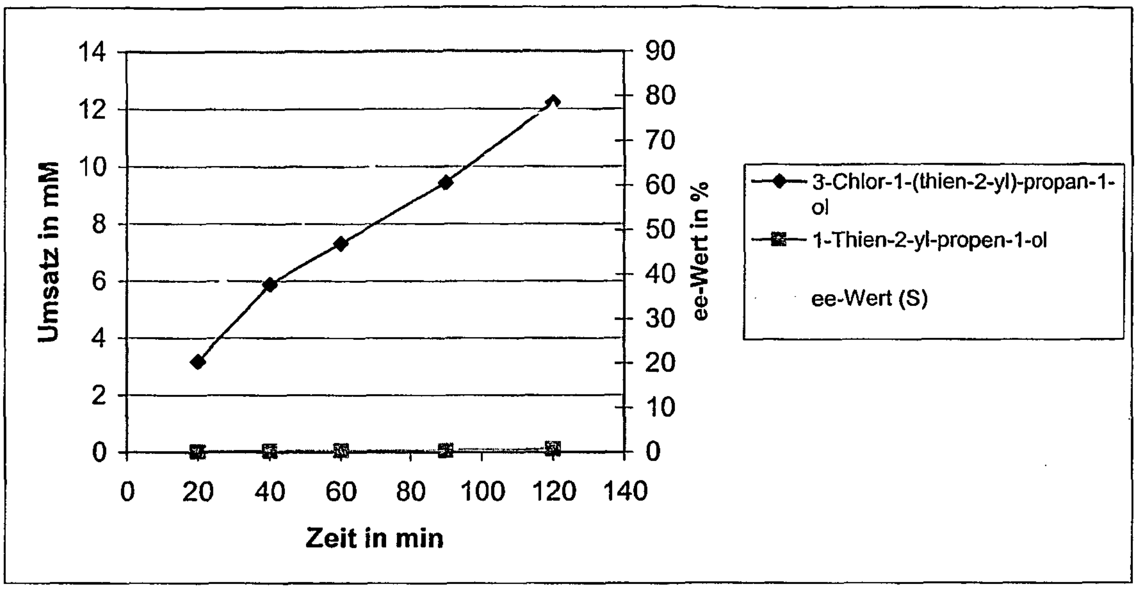
14
12
10
8
6
4
2
0
Umsatz in mM
90
80
70
60
50
40
30
20
10
0
ee-Wert in %
0
20
40
60
80
100
120
140
Zeit in min
3-Chlor-1-(thien-2-yl)-propan-1-ol
1-Thien-2-yl-propen-1-ol
ee-Wert (S)

Fig. 2B

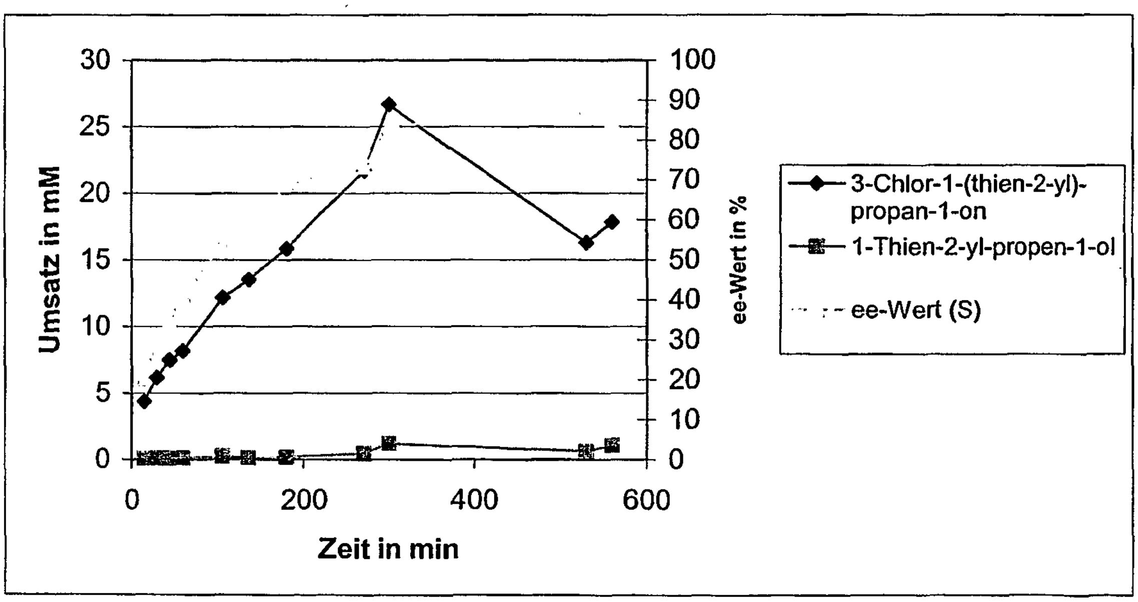
30
25
20
15
10
5
0
Umsatz in mM
100
90
80
70
60
50
40
30
20
10
0
ee-Wert in %
0
200
400
600
Zeit in min
3-Chlor-1-(thien-2-yl)-propan-1-on
1-Thien-2-yl-propen-1-ol
ee-Wert (S)

Fig. 3A

31278/165 Blotbande 1 - LU 10288 (N-terminus)

| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 |
|---|---|---|---|---|---|---|---|---|---|---|---|---|---|---|---|---|---|---|---|---|---|---|---|
| **S** | **N** | **R** | **L** | **D** | **G** | **K** | **V** | **A** | **I** | **V** | **T** | **G** | **G** | **T** | **L** | **G** | **I** | **G** | **L** | **A** | **I** | **A** | **T** |

| 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 |
|---|---|---|---|---|---|---|---|---|---|---|---|---|---|---|---|---|---|---|---|---|---|---|---|
| **F** | **V** | **E** | **E** | **G** | **A** | **K** | **V** | **M** | **I** | **T** | **G** | **R** | **H** | **S** | **D** | **V** | **G** | **E** | **K** | **A** | **A** | **(K)** | **S** |
| | | | | | | | | | | | | | | | | | | | | | **K** | **(A)** | |

| 51 | 52 | 53 | 54 | 55 | 56 | 57 | 58 | 59 | 60 |
|---|---|---|---|---|---|---|---|---|---|
| **G** | **T** | **P** | **D** | **Q** | **I** | **Q** | **F** | **F** | **/** |
| | | **T** | | | | | | **V** | **/** |
| | | | | | | | | **G** | **/** |
| | | | | | | | | **N** | **/** |

# Fig. 3B

## 1. Tryptischer Flüssigverdau Fr. 34 WP Mono P 31279/170

### Tr-Sp-RPFr. 4

| 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|
| T | P | L | V | D | D | L | P | G | A | E | E | A | M | S | Q | R | (R) | / | / |
| S | V | G | T | P | E | Q | I | Q | F | F | Q | H | D | R | | | | | |
| F | A | T | | S | I | F | V | K | | | | Y | T | | | | | | |
| | | | | | | A | | P | | | | | | | | | | | |

### Tr.-Sp.-RP Fr. 6

| 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|
| S | V | E | E | T | T | T | A | E | (W) | R | / | / | / | / |

### Tr.-Sp.-RP Fr. 18

| 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|
| S | V | G | T | P | D | Q | J | Q | F | F | Q | H | D | S | S | D | E | D | G |

### Tr.-Sp.-RP Fr. 4 (3/4 von Filter)

| 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|
| V | N | T | V | H | P | G | Y | J | K | / | / | / | / | / |
| L | F | D | A | T | E | K | / | / | / | / | / | / | / | / |

### Tr.-Sp.-RP Fr. 4 (1/4 von Filter)

| 1 | 2 | 3 | 4 |
|---|---|---|---|
| V | N | T | V |
| L | F | D | A |

### Tr.-RP Fr. 3

| 1 | 2 | 3 | 4 | 5 | 6 | 7 |
|---|---|---|---|---|---|---|
| A | E | I | P | G | K | R |
| V | M | | Q | Y | | N |
| I | | | | | | |
| L,S | | | | | | |

**Tr.-Sp.-RP Fr. 8**

| 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|
| (S) | A | A | L | D | / | A | L | K | / | / | / | / | / | / |

**Tr.-Sp.-RP Fr. 23**

| 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|
| S | A | A | L | D | / | A | L | K | D | Y | / | V | R | / | / | / | / |
| F | (A) | T | G | S | E | F | V | V | (D) | G | G | Y | T | A | Q | / | / |

**Tr.-Sp.-RP Fr. 14**

| 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|
| S | A | A | L | D | / | A | L | K | / | / | / | / | / | / | / | / |

**Tr.-Sp.-RP Fr. 31**

| 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|
| K | L | L | A | V | N | L | D | G | V | F | F | G | T | R | / | / | / | / | / |

**Tr.-Sp.-RP Fr. 2**

| 1 | 2 | 3 | 4 | 5 | 6 |
|---|---|---|---|---|---|
| / | M | / | T | G | R |

**Tr.-Sp.-RP Fr. 27**

| 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|
| I | K | I | P | M | G | H | I | / | E | P | N | / | I | A |
| S | A | A | L | D | G | A | L | K | D | Y | D | V | R | / |
| F | A | T | G | S | E | F | V | V | D | G | G | Y | T | A |

**Tr.-Sp.-RP Fr. 28**

| 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|
| I | K | I | P | M | G | ? | I | (A) | E | (P) | (N) | (D) | I | A | Y | / | / | / | / |
| F | A | T | G | S | E | F | V | V | D | G | G | Y | T | A | Q | | | | |
| S | A | A | L | D | (C) | A | L | K | D | Y | D | V | R | / | | | | | |

## 2. V8-Flüssigverdau Fr.34 WP Mono P 31279/170

### V8-RPFr. 30

| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 |
|---|---|---|---|---|---|---|---|---|---|---|---|---|---|---|---|---|---|---|---|---|---|---|---|
| K | A | A | K | S | V | G | T | P | D | Q | I | Q | F | F | Q | H | D | S | S | P | E | (V) | (V) |
| T | G | L | F | L | | V | L | R | V | A | T | V | | P | G | Y | I | K | A | T | | A | N |
| Q | | | | R | | | V | | N | E | | | | | | | | D | | | | | |

### V8-RPFr. 19

| 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|
| / | V | K | L | L | A | V | N | L | / | / | / | / | / | / | / | / | / | / | / |
| | K | | | A | | | | | I | | | | | | | | | | |
| | R | | | | | | | | D | | | | | | | | | | |

### V8-RPFr. 18

| 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|
| T/G | V | F | F | (G) | L | K | Q | N | I | E | N | I | N | I/N | A | A | V | R | (P) |
| V | | V | N | T | V | H | P | G | Y | I | K | T | P | L | M | D | D | D | |
| | | | | | | | | | | | D | | | | G | | | L | |
| | | | | | | | | | | | | | | | V | | | | |

### V8-RPFr. 22

| 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|
| G | F | V | G | D | P | S | L | G | A | Y | N | A | G | K | G | A | V | R | I |
| Q | V | L | | A | Q | N | M | K | N | G | G | L | S | A | M | I | K | N | M |
| T | S | | | I | Y | R | A | | K | K | A | V | R | I | S | | | | A |
| P | | | | | | K | | | | | | | | | | | | | |
| 21 | 22 | 23 | 24 | 25 | 26 | 27 | 28 | 29 | 30 | | | | | | | | | | |
| M | S | K | S | A | A | L | (D) | / | / | | | | | | | | | | |

### V8-RP Fr. 4

| 1 | 2 | 3 | 4 | 5 | 6 | 7 | 8 | 9 | 10 |
|---|---|---|---|---|---|---|---|---|---|
| F | V | V | D | / | / | / | / | / | / |

**V8-RPFr. 26**

| 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|
| D | G | / | T | K | L | E | D | A | T | E | (E) | / | / | / | / | / | / | / | / |
| R | K | (L) | K | D | A | | E | D | | | | | | | | | | | |
| | Y | (F) | | P | | | | | | | | | | | | | | | |

**V8-RP Fr. 7 (C-Terminus)**

| 1 | 2 | 3 | 4 | 5 | 6 | 7 | 8 | 9 | 10 | 11 | 12 | 13 | 14 |
|---|---|---|---|---|---|---|---|---|---|---|---|---|---|
| F | V | V | D | G | G | Y | T | A | Q | / | / | / | / |

**V8-RP Fr. 28**

| 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|
| / | A | L | K | D | Y | D | V | R | V | N | I | V | H | P | G | Y | I | K | T |
| | S | | | | | | | | | | | | | | | | | | |

| 21 | 22 | 23 | 24 | 25 | 26 | 27 | 28 | 29 | 30 |
|---|---|---|---|---|---|---|---|---|---|
| P | L | V | (V) | D | L | P | G | A | E |
| | | | D | | | | | | (G) |

**V8-RP Fr. 9**

| 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|
| K | A | A | K | S | V | G | T | P | D | Q | I | Q | F | F |

**V8-RP Fr. 2**

| 1 | 2 | 3 | 4 | 5 | 6 | 7 | 8 | 9 | 10 | 11 | 12 | 13 |
|---|---|---|---|---|---|---|---|---|---|---|---|---|
| G | A | K | V | M | I | T | G | R | H | S | D | V |

**V8-RP Fr. 12**

| 1 | 2 | 3 | 4 | 5 | 6 | 7 | 8 | 9 | 10 |
|---|---|---|---|---|---|---|---|---|---|
| S | K | F | A | T | G | S | E | F | V |
| A | | G | | F | V | Y | L | | |
| | | | | I | | | | | |

**V8-RP Fr. 21**

| 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|
| / | D | V | R | V | N | T | V | H | P | G | Y | I | K | T | P | L | V | D | D |
| | V | F | K | I | | | | | | | | | G | A | | | | N | M |
| | S | L | G | A | | | | | | | | | | | | | | | |
| | L | | | G | | | | | | | | | | | | | | | |

| 21 | 22 | 23 | 24 | 25 |
|---|---|---|---|---|
| L | P | G | A | E |
| | L | | | |

**V8-RP Fr. 41**

| 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|
| W | / | K | L | L | A | V | N | L | D | G | V | F | F | G | T | R | L | G | I |

| 21 | 22 | 23 | 24 | 25 | 26 | 27 | 28 | 29 | 30 | 31 | 32 | 33 | 34 | 35 | 36 | 37 | 38 | 39 | 40 |
|---|---|---|---|---|---|---|---|---|---|---|---|---|---|---|---|---|---|---|---|
| Q | R | M | K | N | K | G | L | G | A | S | I | I | N | M | (S) | (S) | (I) | / | / |

**V8-RP Fr. 43**

| 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|
| (A) | M | S | Q | R | T | K | T | P | M | G | H | I | (G) | E | P | N | D | I | A |
| | | K | L | L | A | V | N | L | D | G | V | F | F | G | T | R | L | G | I |
| | | A | K | S | V | G | | | A | Q | I | A | | | | | | | |
| | | | E | | | | | | | Y | N | Q | | | | | | | |

| 21 | 22 | 23 | 24 | 25 | 26 | 27 | 28 | 29 | 30 | 31 | 32 | 33 | 34 | 35 | 36 | 37 | 38 | 39 | 40 |
|---|---|---|---|---|---|---|---|---|---|---|---|---|---|---|---|---|---|---|---|
| Y | | | | Y | | A | | | | | | | | | | | | | |
| Q | R | M | K | N | K | G | L | G | A | S | I | I | N | M | S | | | | G |

**V8-RP Fr. 11**

| 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|
| S | K | F | A | T | G | S | E | F | V | V | / | / | / | / |

**V8-RP Fr. 15**

| 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|
| S | K | E | A | T | G | S | E | F | V | V | D | / | / | / |
| A | | | T | I | L | Q | I | Q | | L | Q | | | |
| F | | | | P | D | | | | | S | K | | | |
| V | | | | | | | | | | | | | | |

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0273658 B
- US 5362886 A
- EP 0457559 A
- EP 1149849 A
- EP 1069183 A
- DE OS100193773 A
- DE 19848129
- WO 200140450 A


### In der Beschreibung aufgeführte Nicht-Patentliteratur


- **W. J. Wheeler ; F. Kuo.** *Journal of Labelled Compounds and Radiopharmaceuticals,* vol. XXXVI (3), 213-223
- **A. Etienne et al.** *CR Acad. Sci., Ser. C,* 1979, vol. 288 (1), 49-52
- **Liu et al.** *Chirality,* 2000, vol. 12, 26-29
- **Meth-Cohn et al.** *Acta Chem. Scand. B,* 1966, vol. 20 (6), 1577-1587
- **Kamal, G. B. ; R. Khanna ; R. Ramu ; T. Krishnaji.** *Tetrahedron Lett.,* 2003, vol. 44, 4783-4787
- Organikum. VEB Deutscher Verlag, 1988, 323 ff
- Organikum. VEB Deutscher Verlag, 1988, 492 ff
- Organikum. VEB Deutscher Verlag, 1988, 486 ff
- **M. Beller ; C. Bolm.** Transition Metals in Organic Synthesis. Wiley-VCH, 1998, vol. 2, 1 ff
- **E. J. Corey ; C. J. Helal.** *Angew. Chem.,* 1998, vol. 110, 2092-2118
- **M. M. Midland ; L. A. Morell.** Methoden Org. Chem. 1995, vol. E 21 d, 4082-4098
- **H. Brunner.** Methoden Org. Chem. 1995, vol. E21d, 4074-4081
- **A. Baiker ; H. U. Blaser.** Handbook of Heterogeneous Catalysis. Wiley-VCH, 1997, vol. 5, 2422-2436
- **R. Noyori ; T. Ohkuma.** *Angew. Chem.,* 2001, vol. 113, 40-75
- **E. J. Corey.** *Pure Appl. Chem.,* 1990, vol. 62, 1209
- **E. J. Corey ; J. O. Link.** *J. Am. Chem. Soc.,* 1992, vol. 114, 1906
- Reduction of Ketones. **K. Nakamura ; T. Matsuda ; K. Drauz ; H. Waldmann.** Enzyme Catalysis in Organic Synthesis. Wiley-VCH, 2002, vol. III, 991-1032
- **W. Hummel ; K. Abokitse ; K. Drauz ; C. Rollmann ; H. Gröger.** *Adv. Synth. Catal.,* 2003, vol. 345 (1, 2), 153-159
- Oxidation of Alcohols. **A. Schmidt ; F. Hollmann ; B. Bühler.** Enzyme Catalysis in Organic Synthesis. Wiley-VCH, 2002, vol. III, 991-1032
- Immobilization of Enzymes. **J. Lalonde ; A. Margolin ; K. Drauz ; H. Waldmann.** Enzyme Catalysis in Organic Synthesis. Wiley-VCH, 2002, vol. III, 991-1032
- **Pearson ; Lipman.** *Proc. Natl. Acad, Sci. (USA),* 1988, vol. 85 (8), 2444-2448
- **Narang, S.A.** *Tetrahedron,* 1983, vol. 39, 3
- **Itakura et al.** *Annu. Rev. Biochem.,* 1984, vol. 53, 323
- **Itakura et al.** *Science,* 1984, vol. 198, 1056
- **Ike et al.** *Nucleic Acids Res.,* 1983, vol. 11, 477
- **Arkin ; Yourvan.** *PNAS,* 1992, vol. 89, 7811-7815
- **Delgrave et al.** *Protein Engineering,* 1993, vol. 6 (3), 327-331
- **Voet ; Voet.** Phosphoamiditmethode. Wiley Press, 896-897
- **Sambrook et al.** Molecular Cloning: A laboratory manual. Cold Spring Harbor Laboratory Press, 1989
- **Sambrook, J. ; Fritsch, E.F. ; Maniatis, T.** Molecular Cloning: A Laboratory Manual. Cold Spring Harbor Laboratory Press, 1989
- **Sambrook et al.** Molecular Cloning. Cold Spring Harbor Laboratory, 1989
- Current Protocols in Molecular Biology. John Wiley & Sons, 1985
- Nucleic Acids Hybridization: A Practical Approach. IRL Press at Oxford University Press, 1985
- Essential Molecular Biology: A Practical Approach. IRL Press at Oxford University Press, 1991
- **Ausubel et al.** Current Protocols in Molecular Biology. John Wiley & Sons, 1989, 6.3.1-6.3.6
- **Goeddel.** Gene Expression Technology: Methods in Enzymology. Academic Press, 1990, vol. 185
- Cloning Vectors. Elsevier, 1985

- **T. Maniatis ; E.F. Fritsch ; J. Sambrook.** Molecular Cloning: A Laboratory Manual. Cold Spring Harbor Laboratory, 1989
- **T.J. Silhavy ; M.L. Berman ; L.W. Enquist.** Experiments with Gene Fusions. Cold Spring Harbor Laboratory, 1984
- **Ausubel, F.M. et al.** Current Protocols in Molecular Biology. Greene Publishing Assoc. and Wiley Interscience, 1987
- Current Protocols in Molecular Biology. Wiley Interscience, 1997
- **Sambrook et al.** Molecular Cloning: A Laboratory Manual. Cold Spring Harbor Laboratory Press, 1989
- **Thomas, K.R. ; Capecchi, M.R.** *Cell,* 1987, vol. 51, 503
- **Cooper, F. G.** Biochemische Arbeitsmethoden. Verlag Walter de Gruyter
- **Scopes, R.** Protein Purification. Springer Verlag
- **Harlow, E. ; Lane, D.** Antibodies: A Laboratory Manual. Cold Spring Harbor (N.Y.) Press, 1988
- Biotechnology. 1993, vol. 3
- **Takeshita, S ; Sato, M ; Toba, M ; Masahashi, W ; Hashimoto-Gotoh, T.** *Gene,* 1987, vol. 61, 63-74
- **T. Tomoyasu et al.** *Mol. Microbiol.,* 2001, vol. 40 (2), 397-413

Sequenzprotokoll

<110> BASF Aktiengesellschaft

<120> Verfahren zur Herstellung von 3-Methylamino-1-(thien-2-yl)-propan-1-ol

<130> M/44142

<160> 6

<170> PatentIn version 3.1

<210> 1
<211> 47
<212> PRT
<213> Lactobacillus brevis

<400> 1

Met Ser Asn Arg Leu Asp Gly Lys Val Ala Ile Val Thr Gly Gly Thr
1 5 10 15

Leu Gly Ile Gly Leu Ala Ile Ala Thr Lys Phe Val Glu Glu Gly Ala
20 25 30

Lys Val Met Ile Thr Gly Arg His Ser Asp Val Gly Glu Lys Ala
35 40 45

<210> 2
<211> 18
<212> PRT
<213> Candida magnoliae

<400> 2

Ser Asn Ala Leu Val Thr Gly Gly Ser Arg Val Ile Gly Ala Gly Gly
1 5 10 15

Phe Ile

<210> 3
<211> 756
<212> DNA
<213> Lactobacillus brevis

<220>
<221> CDS
<222> (1)..(756)
<223>

<400> 3

```
atg tct aac cgt ttg gat gga aaa gta gca atc gtt aca ggt ggt acg       48
Met Ser Asn Arg Leu Asp Gly Lys Val Ala Ile Val Thr Gly Gly Thr
1               5                   10                  15

ttg ggt atc ggt tta gct atc gcc acg aag ttc gtt gaa gaa ggg gct       96
Leu Gly Ile Gly Leu Ala Ile Ala Thr Lys Phe Val Glu Glu Gly Ala
            20                  25                  30

aag gtc atg att acc ggc cgg cac agc gat gtt ggt gaa aaa gca gct      144
Lys Val Met Ile Thr Gly Arg His Ser Asp Val Gly Glu Lys Ala Ala
        35                  40                  45

aag agt gtc ggc act cct gat cag att caa ttt ttc caa cat gat tct      192
Lys Ser Val Gly Thr Pro Asp Gln Ile Gln Phe Phe Gln His Asp Ser
    50                  55                  60

tcc gat gaa gac ggc tgg acg aaa tta ttc gat gca acg gaa aaa gcc      240
Ser Asp Glu Asp Gly Trp Thr Lys Leu Phe Asp Ala Thr Glu Lys Ala
65                  70                  75                  80

ttt ggc cca gtt tct aca tta gtt aat aac gct ggg atc gcg gtt aac      288
Phe Gly Pro Val Ser Thr Leu Val Asn Asn Ala Gly Ile Ala Val Asn
                85                  90                  95

aag agt gtc gaa gaa acc acg act gct gaa tgg cgt aaa cta tta gcc      336
Lys Ser Val Glu Glu Thr Thr Thr Ala Glu Trp Arg Lys Leu Leu Ala
            100                 105                 110

gtc aac ctt gat ggt gtc ttc ttc ggt acc cga tta ggg att caa cgg      384
```

```
Val Asn Leu Asp Gly Val Phe Phe Gly Thr Arg Leu Gly Ile Gln Arg
        115                 120                     125

atg aag aac aaa ggc tta ggg gct tcc atc atc aac atg tct tcg atc      432
Met Lys Asn Lys Gly Leu Gly Ala Ser Ile Ile Asn Met Ser Ser Ile
    130                 135                     140

gaa ggc ttt gtg ggt gat cct agc tta ggg gct tac aac gca tct aaa      480
Glu Gly Phe Val Gly Asp Pro Ser Leu Gly Ala Tyr Asn Ala Ser Lys
145                 150                 155                 160

ggg gcc gta cgg att atg tcc aag tca gct gcc tta gat tgt gcc cta      528
Gly Ala Val Arg Ile Met Ser Lys Ser Ala Ala Leu Asp Cys Ala Leu
                165                 170                 175

aag gac tac gat gtt cgg gta aac act gtt cac cct ggc tac atc aag      576
Lys Asp Tyr Asp Val Arg Val Asn Thr Val His Pro Gly Tyr Ile Lys
            180                 185                 190

aca cca ttg gtt gat gac cta cca ggg gcc gaa gaa gcg atg tca caa      624
Thr Pro Leu Val Asp Asp Leu Pro Gly Ala Glu Glu Ala Met Ser Gln
        195                 200                 205

cgg acc aag acg cca atg ggc cat atc ggt gaa cct aac gat att gcc      672
Arg Thr Lys Thr Pro Met Gly His Ile Gly Glu Pro Asn Asp Ile Ala
    210                 215                 220

tac atc tgt gtt tac ttg gct tct aac gaa tct aaa ttt gca acg ggt      720
Tyr Ile Cys Val Tyr Leu Ala Ser Asn Glu Ser Lys Phe Ala Thr Gly
225                 230                 235                 240

tct gaa ttt gta gtt gac ggt ggc tac act gct caa                      756
Ser Glu Phe Val Val Asp Gly Gly Tyr Thr Ala Gln
                245                 250
```

<210> 4
<211> 252
<212> PRT
<213> Lactobacillus brevis

<400> 4

```
Met Ser Asn Arg Leu Asp Gly Lys Val Ala Ile Val Thr Gly Gly Thr
1               5                   10                  15

Leu Gly Ile Gly Leu Ala Ile Ala Thr Lys Phe Val Glu Glu Gly Ala
            20                  25                  30

Lys Val Met Ile Thr Gly Arg His Ser Asp Val Gly Glu Lys Ala Ala
        35                  40                  45
```

```
Lys Ser Val Gly Thr Pro Asp Gln Ile Gln Phe Phe Gln His Asp Ser
    50                  55                  60

Ser Asp Glu Asp Gly Trp Thr Lys Leu Phe Asp Ala Thr Glu Lys Ala
65                  70                  75                  80

Phe Gly Pro Val Ser Thr Leu Val Asn Asn Ala Gly Ile Ala Val Asn
                85                  90                  95

Lys Ser Val Glu Glu Thr Thr Thr Ala Glu Trp Arg Lys Leu Leu Ala
            100                 105                 110

Val Asn Leu Asp Gly Val Phe Phe Gly Thr Arg Leu Gly Ile Gln Arg
        115                 120                 125

Met Lys Asn Lys Gly Leu Gly Ala Ser Ile Ile Asn Met Ser Ser Ile
    130                 135                 140

Glu Gly Phe Val Gly Asp Pro Ser Leu Gly Ala Tyr Asn Ala Ser Lys
145                 150                 155                 160

Gly Ala Val Arg Ile Met Ser Lys Ser Ala Ala Leu Asp Cys Ala Leu
                165                 170                 175

Lys Asp Tyr Asp Val Arg Val Asn Thr Val His Pro Gly Tyr Ile Lys
            180                 185                 190

Thr Pro Leu Val Asp Asp Leu Pro Gly Ala Glu Glu Ala Met Ser Gln
        195                 200                 205

Arg Thr Lys Thr Pro Met Gly His Ile Gly Glu Pro Asn Asp Ile Ala
    210                 215                 220

Tyr Ile Cys Val Tyr Leu Ala Ser Asn Glu Ser Lys Phe Ala Thr Gly
225                 230                 235                 240

Ser Glu Phe Val Val Asp Gly Gly Tyr Thr Ala Gln
                245                 250
```

<210> 5
<211> 472
<212> DNA
<213> Candida magnoliae

<220>
<221> CDS

<222> (1)..(471)
<223>

<400> 5

```
aac gcg ctg gtg acg ggc ggc agc cgc ggc att ggc gaa gcc act gcc        48
Asn Ala Leu Val Thr Gly Gly Ser Arg Gly Ile Gly Glu Ala Thr Ala
1               5                   10                  15

att aag ctc gcc gag gag ggc tac agc gtc acg att gcg tct cgc ggc        96
Ile Lys Leu Ala Glu Glu Gly Tyr Ser Val Thr Ile Ala Ser Arg Gly
            20                  25                  30

ctt aag cag ctc gag gct gtg aag gcc aaa cta ccc att gtg aag cag       144
Leu Lys Gln Leu Glu Ala Val Lys Ala Lys Leu Pro Ile Val Lys Gln
        35                  40                  45

gga cag gtt cac cac gtg tgg cag ctt gat ctc agt gat gtc gac gct       192
Gly Gln Val His His Val Trp Gln Leu Asp Leu Ser Asp Val Asp Ala
    50                  55                  60

gcg gcc gcc ttc aaa ggg tcg ccg cta cct gcc agc cgc tac gac gtg       240
Ala Ala Ala Phe Lys Gly Ser Pro Leu Pro Ala Ser Arg Tyr Asp Val
65                  70                  75                  80

ctc gtc agc aat gct ggc gtg gcc cag ttt agc ccg ttc atc gag cat       288
Leu Val Ser Asn Ala Gly Val Ala Gln Phe Ser Pro Phe Ile Glu His
                85                  90                  95

gcg aag cag gac tgg tcg cag atg ctt gcc atc aat ctg gcg gca ccc       336
Ala Lys Gln Asp Trp Ser Gln Met Leu Ala Ile Asn Leu Ala Ala Pro
            100                 105                 110

att gcg ctg gcc cag aca ttt gct aag gcc att ggc gac aag ccg cgc       384
Ile Ala Leu Ala Gln Thr Phe Ala Lys Ala Ile Gly Asp Lys Pro Arg
        115                 120                 125

aac aca ccg gcc cac att gtg ttt gtc tcg tcg aac gtc tcg ttg cga       432
Asn Thr Pro Ala His Ile Val Phe Val Ser Ser Asn Val Ser Leu Arg
    130                 135                 140

ggc ttc ccg aac atc ggc gtc aac tcc atc acc ccc ggc a                 472
Gly Phe Pro Asn Ile Gly Val Asn Ser Ile Thr Pro Gly
145                 150                 155
```

<210> 6
<211> 157
<212> PRT
<213> Candida magnoliae

<400> 6

```
Asn Ala Leu Val Thr Gly Gly Ser Arg Gly Ile Gly Glu Ala Thr Ala
1               5                   10                  15

Ile Lys Leu Ala Glu Glu Gly Tyr Ser Val Thr Ile Ala Ser Arg Gly
            20                  25                  30

Leu Lys Gln Leu Glu Ala Val Lys Ala Lys Leu Pro Ile Val Lys Gln
        35                  40                  45

Gly Gln Val His His Val Trp Gln Leu Asp Leu Ser Asp Val Asp Ala
    50                  55                  60

Ala Ala Ala Phe Lys Gly Ser Pro Leu Pro Ala Ser Arg Tyr Asp Val
65                  70                  75                  80

Leu Val Ser Asn Ala Gly Val Ala Gln Phe Ser Pro Phe Ile Glu His
                85                  90                  95

Ala Lys Gln Asp Trp Ser Gln Met Leu Ala Ile Asn Leu Ala Ala Pro
            100                 105                 110

Ile Ala Leu Ala Gln Thr Phe Ala Lys Ala Ile Gly Asp Lys Pro Arg
        115                 120                 125

Asn Thr Pro Ala His Ile Val Phe Val Ser Ser Asn Val Ser Leu Arg
    130                 135                 140

Gly Phe Pro Asn Ile Gly Val Asn Ser Ile Thr Pro Gly
145                 150                 155
```